# FASCICULE DE BREVET EUROPEEN

(11) **EP 1 937 672 B1**
(45) Date de publication et mention de la délivrance du brevet: **02.09.2009**
(21) Numéro de dépôt: 06778797.8
(22) Date de dépôt: 06.07.2006
(51) Int. Cl.: C07D 409/12, C07D 405/12, C07D 403/12, C07D 239/26, A61K 31/506, A61P 25/00, A61P 3/00, A61P 1/00

(54) **DERIVES DE N-[(4,5-DIPHENYLPYRIMIDIN-2-YL)METHYL]AMINE, LEUR PREPARATION ET LEUR APPLICATION EN THERAPEUTIQUE**
N-[(4,5-DIPHENYLPYRIMIDIN-2-YL)METHYL]AMINDERIVATE, DEREN HERSTELLUNG UND DEREN THERAPEUTISCHE VERWENDUNG
N-[(4,5-DIPHENYLPYRIMIDIN-2-YL)METHYL]AMINE DERIVATIVES, THE PREPARATION THEREOF AND THEIR THERAPEUTIC USE

(30) Priorité: 08.07.2005 FR 0507359
(43) Date de publication de la demande: 02.07.2008
(73) Titulaire: Sanofi-Aventis, 75013 Paris (FR)
(72) Inventeur: BARTH, Francis, 34680 Saint-Georges d'Orques (FR); CONGY, Christian, F-34980 Saint Gely du Fesc (FR); GUEULE, Patrick, F-34820 Teyran (FR); RINALDI-CARMONA, Murielle, F-34680 Saint Georges d'Orques (FR)
(74) Mandataire: Kugel, Dominique
(86) Numéro de dépôt international: PCT/FR2006/001621
(87) Numéro de publication internationale: WO 2007/006928

(56) Documents cités:
- WO-A-92/02513
- WO-A-20/04029204
- WO-A-20/04110453

## Description

La présente invention a pour objet des dérivés de N-[(4,5-diphénylpyrimidin-2-yl)méthyl]amine substitués, leur préparation et leur application en thérapeutique.

Des dérivés de diphénylpyrazole présentant une affinité pour les récepteurs CB₁ des cannabinoïdes ont été décrits notamment dans les brevets US 5 624 941, EP 0 576 357, EP 0 656 354, EP 1 150 961.

Des dérivés de 4,5-diarylpyrimidine comme ligands des récepteurs aux cannabinoïdes sont décrits dans la demande internationale WO 2004/110453 ou dans la demande internationale WO 2004/029204.

Des dérivés de 4,5-bis[(C₁-C₆)alcoxyphényl]pyrimidine possédant des activités antithrombotiques, vasodilatatoires et antiinflammatoires sont décrits dans la demande internationale WO 92/02513.

On a maintenant trouvé des nouveaux dérivés de N-[(4,5-diphénylpyrimidin-2-yl)méthyl]amine substitués qui possèdent des propriétés antagonistes des récepteurs CB₁ des cannabinoïdes, localisés au niveau central et/ou périphérique.

La présente invention a pour objet des composés répondant à la formule : 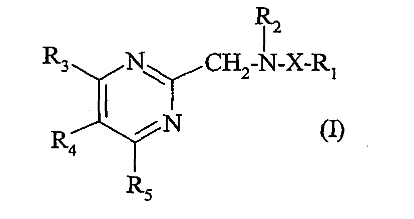 dans laquelle :
- X représente un groupe 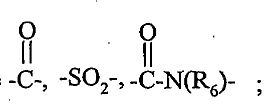
- R₁ représente :
   - un (C₁-C₁₂)alkyle non substitué ou substitué une ou plusieurs fois par des substituants choisis indépendamment parmi un atome de fluor, un hydroxyle, un (C₁-C₄) alcoxy, un (C₁-C₄)alkylthio, un phénoxy, un radical trifluorométhoxy, un radical difluorométhoxy, un radical difluorométhylthio, un radical trifluorométhylthio ;
   - un radical carbocyclique non aromatique en (C₃-C₁₂) non substitué ou substitué une ou plusieurs fois par des substituants choisis indépendamment parmi un (C₁-C₄)alkyle, un (C₁-C₄)alcoxy, un atome de fluor, un hydroxyle, un radical trifluorométhyle, un radical trifluorométhoxy, un (C₁-C₄)alkylthio ;
   - un (C₃-C₇)cycloalkylméthyle non substitué ou substitué une ou plusieurs fois sur le carbocycle par des substituants choisis indépendamment parmi un (C₁-C₄)alkyle, un (C₁-C₄)alcoxy, un atome de fluor, un hydroxyle, un radical trifluorométhyle, un radical trifluorométhoxy ;
   - un phényle non substitué ou substitué une ou plusieurs fois par des substituants choisis indépendamment parmi un atome d'halogène, un hydroxyle, un groupe Alk, un groupe OAlk, un méthylènedioxy, un (C₁-C₄)alkylamino, un di-(C₁-C₄)alkylamino, un cyano, un nitro, un groupe S(O)ₙAlk, un groupe OS(O)ₙAlk, un groupe (C₁-C₄)alkylcarbonyle ; ou parmi un radical phényle, phénoxy, pyrrolyle, imidazolyle, pyridyle ou pyrazolyle, ledit radical étant non substitué ou substitué une ou plusieurs fois par un (C₁-C₄)alkyle ;
   - un benzyle non substitué ou substitué une ou plusieurs fois sur le phényle par des substituants choisis indépendamment parmi un atome d'halogène, un groupe Alk, un hydroxyle, un groupe OAlk, un méthylènedioxy, un groupe S(O)ₙAlk, un groupe OS(O)ₙAlk et non substitué ou substitué en alpha par un ou deux groupes semblables ou différents choisis parmi un (C₁-C₄)alkyle, un (C₃-C₇)cycloalkyle ;
   - un phénéthyle non substitué ou substitué une ou plusieurs fois sur le phényle par des substituants choisis indépendamment parmi un atome d'halogène, un (C₁-C₄)alkyle, un (C₁-C₄)alcoxy, un radical trifluorométhyle, un radical trifluorométhoxy ;
   - un benzhydryle ; un benzhydrylméthyle ;
   - un 1,2,3,4-tétrahydronaphtalènyle non substitué ou substitué une ou plusieurs fois par un (C₁-C₄)alkyle ;
   - un radical hétérocyclique aromatique choisi parmi un pyrrolyle, un imidazolyle, un furyle, un thiényle, un pyrazolyle, un oxazolyle, un pyridyle, un indolyle, ledit radical étant non substitué ou substitué une ou plusieurs fois par des substituants choisis indépendamment parmi un atome d'halogène ou un (C₁-C₄)alkyle, un (C₁-C₄)alcoxy, un radical trifluorométhyle, un radical trifluorométhoxy, un cyano, un nitro, un (C₁-C₄)alkylthio ;
- R₂ représente un atome d'hydrogène ou un (C₁-C₃)alkyle ;
- R₃ représente un atome d'hydrogène ou un (C₁-C₅)alkyle ;
- R₄ représente un phényle non substitué ou substitué une ou plusieurs fois par des substituants choisis indépendamment parmi un atome d'halogène, un groupe Alk, un groupe OAlk, un groupe S(O)ₙAlk ou un groupe OS(O)ₙAlk ;
- R₅ représente un phényle non substitué ou substitué une ou plusieurs fois par des substituants choisis indépendamment parmi un atome d'halogène, un groupe Alk, un groupe OAlk, un groupe S(O)ₙAlk ou un groupe OS(O)ₙAlk ;
- R₆ représente un atome d'hydrogène ou un (C₁-C₃)alkyle ;
- n représente 0, 1 ou 2 ;
- Alk représente un (C₁-C₄)alkyle non substitué ou substitué une ou plusieurs fois par un atome de fluor ;
   à la condition que R₄ et R₅ ne représentent pas simultanément un phényle substitué par un (C₁-C₄)alcoxy.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères ainsi que leurs mélanges, y compris les mélanges racémiques, font partie de l'invention.

Les composés de formule (I) peuvent exister sous forme d'hydrates ou de solvats, à savoir sous forme d'associations ou de combinaisons avec une ou plusieurs molécules d'eau ou avec un solvant. De tels hydrates et solvats font également partie de l'invention.

Par atome d'halogène on entend un atome de brome, de chlore, de fluor ou d'iode.

Par (C₁-C₃)alkyle ou respectivement (C₁-C₄)alkyle, (C₁-C₅)alkyle ou (C₁-C₁₂)alkyle, on entend un radical alkyle linéaire ou ramifié de un à trois atomes de carbone ou respectivement de un à quatre atomes de carbone, de un à cinq atomes de carbone ou de un à douze atomes de carbone, tel que le radical méthyle, éthyle, propyle, isopropyle, butyle, isobutyle, *sec*-butyle, *tert*-butyle, pentyle, isopentyle, hexyle, isohexyle, heptyle, octyle, nonyle, décyle, undécycle, dodécyle.

Par (C₁-C₄)alcoxy on entend un radical alcoxy linéaire ou ramifié de un à quatre atomes de carbone tel que le radical méthoxy, éthoxy, propoxy, isopropoxy, butoxy, sec-butoxy, *tert*-butoxy.

Par (C₃-C₇)cycloalkyle on entend un groupe alkyle cyclique de 3 à 7 atomes de carbone, tel que le groupe cyclopropyle, cyclobutyle, cyclopentyle, cyclohexyle, cycloheptyle.

Les radicaux carbocycliques non aromatiques en C₃-C₁₂ comprennent les radicaux mono ou polycycliques, condensés, pontés ou spiraniques. Les radicaux monocycliques incluent les cycloalkyles par exemple cyclopropyle, cyclobutyle, cyclopentyle, cyclohexyle, cycloheptyle, cyclooctyle. Les radicaux di- ou tricycliques condensés, pontés ou spiraniques, incluent par exemple les radicaux norbornyle, bornyle, isobornyle, noradamantyle, adamantyle, spiro[5.5]undécyle, bicyclo[2.2.1] heptyle, bicyclo[3.2.1]octyle ; bicyclo[3.1.1]heptyle.

Parmi les composés de formule (I), objets de l'invention, on distingue les composés de formule (I) dans laquelle :
- X représente un groupe 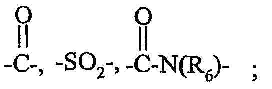
- R₁ représente :
   - un (C₁-C₇)alkyle ;
   - un radical carbocyclique non aromatique en (C₃-C₁₂) non substitué ou substitué une ou plusieurs fois par un (C₁-C₄)alkyle ;
   - un (C₃-C₇)cycloalkylméthyle non substitué ou substitué une ou plusieurs fois sur le carbocycle par un (C₁-C₄)alkyle ;
   - un phényle non substitué ou mono-, di- ou -trisubstitué par des substituants choisis indépendamment parmi un atome d'halogène, un (C₁-C₄)alkyle, un (C₁-C₄)alcoxy, un (C₁-C₄)alkylamino, un di-(C₁-C₄)alkylamino, un cyano, un radical trifluorométhyle, un radical trifluorométhoxy, un groupe S(O)ₙAlk, un groupe (C₁-C₄)alkylcarbonyle ; ou parmi un radical phényle, phénoxy, pyrrolyle, imidazolyle, pyridyle ou pyrazolyle, lesdits radicaux étant non substitués ou substitués une ou plusieurs fois par un (C₁-C₄)alkyle ;
   - un benzyle non substitué ou mono- ou disubstitué sur le phényle par des substituants choisis indépendamment parmi un atome d'halogène, un (C₁-C₄)alkyle, un (C₁-C₄)alcoxy, un radical trifluorométhyle ;
   - un benzhydryle ; un benzhydrylméthyle ;
   - un radical hétérocyclique aromatique choisi parmi un pyrrolyle, un imidazolyle, un furyle, un thiényle, un pyrazolyle, un indolyle non substitué ou substitué une
ou plusieurs fois par des substituants choisis indépendamment parmi un atome d'halogène ou un (C₁-C₄)alkyle ;
- R₂ représente un atome d'hydrogène ou un (C₁-C₃)alkyle ;
- R₃ représente un atome d'hydrogène ou un (C₁-C₅)alkyle ;
- R₄ représente un phényle non substitué ou mono-, di- ou trisubstitué par des substituants choisis indépendamment parmi un atome d'halogène, un (C₁-C₄)alkyle, un (C₁-C₄)alcoxy, un radical trifluorométhyle ou un groupe S(O)ₙAlk ;
- R₅ représente un phényle non substitué ou mono-, di- ou trisubstitué par des substituants choisis indépendamment parmi un atome d'halogène, un (C₁-C₄)alkyle, un (C₁-C₄)alcoxy, un radical trifluorométhyle ou un groupe S(O)ₙAlk ;
- R₆ représente un atome d'hydrogène ou un (C₁-C₃)alkyle ;
- n représente 0, 1 ou 2 ;
- Alk représente un (C₁-C₄)alkyle ; à la condition que R₄ et R₅ ne représentent pas simultanément un phényle substitué par un (C₁-C₄)alcoxy.

Parmi les composés de formule (I), objets de l'invention, on distingue :
- les composés de formule (IA) dans laquelle -X- représente un radical -CO- et les substituants R₁ à R₅ sont tels que définis pour les composés de formule (I) ;
- les composés de formule (IB) dans laquelle -X- représente un radical -SO₂- et les substituants R₁ à R₅ sont tels que définis pour les composés de formule (I) ;
- les composés de formule (IC) dans laquelle -X- représente un radical -CON(R₆)- et les substituants R₁ à R₆ sont tels que définis pour les composés de formule (I).

Parmi les composés de formule (I), objets de l'invention, un premier groupe de composés est constitué par les composés pour lesquels :
- X est tel que défini pour un composé de formule (I) ;
- R₁ représente :
   - un (C₁-C₇)alkyle ;
   - un (C₃-C₇)cycloalkyle ;un norbornyle ;
   - un phényle non substitué ou mono- ou disubstitué par des substituants choisis indépendamment parmi un atome d'halogène, un (C₁-C₄)alkyle, un (C₁-C₄)alcoxy, un di-(C₁-C₄)alkylamino, un nitro, un radical trifluorométhyle, un radical difluorométhoxy, un phényle, un phénoxy, un pyrrolyle ;
   - un benzyle non substitué ou substitué par un radical trifluorométhyle ;
   - un benzhydrylméthyle ;
   - un radical hétérocyclique aromatique choisi parmi un pyrazolyle, un furyle, un thiényle, un indolyle non substitué ou substitué une ou deux fois par des substituants choisis indépendamment parmi un atome d'halogène ou un (C₁-C₄)alkyle ;
- et/ou R₂ représente un atome d'hydrogène ;
- et/ou R₃ représente un atome d'hydrogène ou un méthyle ;
- et/ou R₄ représente un phényle substitué par un atome d'halogène ou un (C₁-C₄)alcoxy ;
- et/ou R₅ représente un phényle mono- ou disubstitué par un atome d'halogène ;
- et/ou R₆ représente un atome d'hydrogène ;
   ainsi que leurs hydrates ou leurs solvats.

Parmi les composés de ce dernier goupe, on peut citer les composés de formule (I) pour lesquels :
- X est tel que défini pour un composé de formule (I) ;
- R₁ représente :
   - un 1-éthylpropyle ; un 1-propylbutyle ;
   - un cyclopentyle ; un cyclohexyle ; un cycloheptyle ; un 2-norbornyle ;
   - un phényle ; un 4-bromophényle ; un 3-chlorophényle ; un 4-chlorophényle ; un 2-fluorophényle ; un 3-fluorophényle ; un 3,5-difluorophényle ; un 4-*tert-*butylphényle ; un 3,5-diméthylphényle ; un 3-méthoxyphényle ; un 4-(diéthylamino)phényle ; un 3-(trifluorométhyl)phényle ; un 4-(trifluorométhyl) phényle ; un 2-chloro-4-(trifluorométhyl)phényle ; un 2-nitro-4-(trifluorométhyl)phényle ; un 2-bromo-4-(trifluorométhyl)phényle ; un 2-fluoro-5-(trifluorométhyl)phényle ; un 4-fluoro-3-(trifluorométhyl)phényle ; un 2-chloro-5-(trifluorométhyl)phényle ; un 4-(difluorométhoxy)phényle ; un biphényl-2-yle ; un 3-phénoxyphényle ;un 4-phénoxyphényle ; un 4-(1*H*-pyrrol-1-yl) phényle ;
   - un 3-(trifluorométhyl)benzyle ;
   - un benzhydrylméthyle ;
   - un 2-thiényle ; un 5-chloro-2-thiényle ; un 5-bromo-2-furyle ; un 3-*tert*-butyl-1-éthyl-1*H*-pyrazol-5-yle ; un 1*H*-indol-2-yle ; un 1-méthyl-1*H*-indol-2-yle ;
- R₂ représente un atome d'hydrogène ;
- R₃ représente un atome d'hydrogène ou un méthyle ;
- R₄ représente un 4-bromophényle ; un 4-chlorophényle ; un 4-méthoxyphényle ;
- R₅ représente un 2,4-dichlorophényle ; un 2-chlorophényle ;
- R₆ représente un atome d'hydrogène ;
   ainsi que leurs hydrates ou leurs solvats.

Parmi les composés de ce dernier groupe, on peut citer les composés de formule (I) pour lesquels :
- X représente un groupe -CO- ou -SO₂- ;
- R₁ représente :
   - un 3-(trifluorométhyl)phényle ; un 4-(trifluorométhyl)phényle ; un 2-chloro-4-(trifluorométhyl)phényle ; un 3-phénoxyphényle ; un 4-(1H-pyrrol-1-yl)phényle ;
   - un benzhydrylméthyle ;
- R₂ représente un atome d'hydrogène ;
- R₃ représente un atome d'hydrogène ou un méthyle ;
- R₄ représente un 4-bromophényle ou un 4-méthoxyphényle ;
- R₅ représente un 2,4-dichlorophényle ; un 2-chlorophényle ;
   ainsi que leurs hydrates ou leurs solvats.

Parmi les composés de formule (I) objets de l'invention, on peut notamment citer les composés suivants :
- N-[[5-(4-bromophényl)-4-(2,4-dichlorophényl)pyrimidin-2-yl]méthyl]-3-(trifluorométhyl)benzènesulfonamide ;
- N-[[5-(4-bromophényl)-4-(2,4-dichlorophényl)pyrimidin-2-yl]méthyl]-4-(trifluorométhyl)benzènesulfonamide ;
- N-[[4-(2,4-dichlorophényl)-5-(4-méthoxyphényl)pyrimidin-2-yl]méthyl]-4-(1*H-*pyrrol-1-yl)benzamide ;
- N-[[5-(4-bromophényl)-4-(2,4-dichlorophényl)pyrimidin-2-yl]méthyl]-3,3-diphénylpropanamide ;
- N-[[5-(4-bromophényl)-4-(2,4-dichlorophényl)-6-méthylpyrimidin-2-yl]méthyl]-3,3-diphénylpropanamide ;
- N-[[5-(4-bromophényl)-4-(2,4-dichlorophényl)-6-méthylpyrimidin-2-yl]méthyl]-3-(trifluorométhyl)benzènesulfonamide ;
- N-[[5-(4-bromophényl)-4-(2,4-dichlorophényl)-6-méthylpyrimidin-2-yl]méthyl]-4-(trifluorométhyl)benzènesulfonamide ;
- N-[[5-(4-bromophényl)-4-(2,4-dichlorophényl)-6-méthylpyrimidin-2-yl]méthyl]-2-chloro-4-(trifluorométhyl)benzènesulfonamide ;
- N-[[5-(4-bromophényl)-4-(2,4-dichlorophényl)-6-méthylpyrimidin-2-yl]méthyl]-3-phénoxybenzènesulfonamide ;
- N-[[5-(4-bromophényl)-4-(2-chlorophényl)pyrimidin-2-yl]méthyl]-4-(trifluorométhyl)benzènesulfonamide ;ainsi que leurs hydrates ou leurs solvats.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans "Advances in Organic Chemistry", J. March, 3rd Edition, Wiley Interscience, 1985, p. 310-316.

Conformément à l'invention, on peut préparer les composés de formule (I) selon un procédé qui est **caractérisé en ce que :**
on traite un composé de formule : 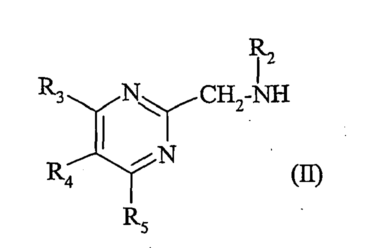
dans laquelle R₂, R₃, R₄ et R₅ sont tels que définis pour un composé de formule (I) :
- soit par un acide ou un dérivé fonctionnel de cet acide de formule :

   HOOC-R₁ (III)
dans laquelle R₁ est tel que défini pour un composé de formule (I), lorsqu'on doit préparer un composé de formule (I) dans laquelle -X- représente un groupe -CO- ;
- soit par un halogénure de sulfonyle de formule :

   Hal-SO₂-R₁ (IV)
dans laquelle R₁ est tel que défini pour un composé de formule (I) et Hal représente un atome d'halogène, préférentiellement le chlore, lorsqu'on doit préparer un composé de formule (I) dans laquelle -X- représente un groupe -SO₂- ;
- soit par un halogénoformiate de formule :

   HalCOOAr (V)

   dans laquelle Hal représente un atome d'halogène et Ar représente un phényle ou un 4-nitrophényle pour obtenir un composé intermédiaire de formule : 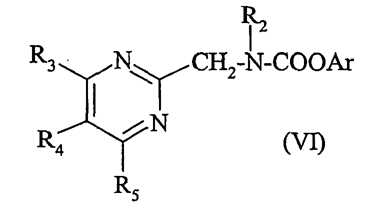 dans laquelle R₂, R₃, R₄ et R₅ sont tels que définis pour un composé de formule (I), que l'on fait réagir ensuite avec une amine de formule :

   HN(R₆)R₁ (VII)

   dans laquelle R₁ et R₆ sont tels que définis pour un composé de formule (I), lorsqu'on doit préparer un composé de formule (I) dans laquelle -X- représente un groupe -CON(R₆)-.

Lorsqu'on traite un composé de formule (II) avec l'acide de formule (III) lui-même, on opère en présence d'un agent de couplage utilisé en chimie peptidique tel que le 1,3-dicyclohexylcarbodiimide ou l'hexafluorophosphate de benzotriazol-1-yloxytris(diméthylamino) phosphonium ou l'hexafluorophosphate de benzotriazol-1-yloxytris(pyrrolidino) phosphonium ou le tétrafluoroborate de 2-(1*H*-benzotriazol-1-yl)-1,1,3,3-tétraméthyl uronium, en présence d'une base telle que la triéthylamine, la N,N-diisopropyléthyl amine ou la 4-diméthylaminopyridine, dans un solvant tel que le dichlorométhane, le dichloroéthane, le N-N-diméthylformamide ou le tétrahydrofurane à une température comprise entre -10°C et la température de reflux du solvant.

Comme dérivé fonctionnel de l'acide (III) on peut utiliser le chlorure d'acide, l'anhydride, un anhydride mixte, un ester alkylique en C₁-C₄ dans lequel l'alkyle est droit ou ramifié, un ester activé, par exemple l'ester de *p*-nitrophényle.

Ainsi dans le procédé selon l'invention, on peut aussi faire réagir le chlorure de l'acide obtenu par réaction du chlorure de thionyle ou du chlorure d'oxalyle sur l'acide de formule (III), avec le composé de formule (II), dans un solvant, tel qu'un solvant chloré (le dichlorométhane, le dichloroéthane, le chloroforme par exemple), un éther (tétrahydrofurane, dioxane par exemple), ou un amide (N,N-diméthylformamide par exemple) sous une atmosphère inerte, à une température comprise entre 0°C et la température ambiante, en présence d'une amine tertiaire telle que la triéthylamine, la N-méthylmorpholine ou la pyridine.

Une variante consiste à préparer l'anhydride mixte de l'acide de formule (III) par réaction du chloroformiate d'éthyle avec l'acide de formule (III), en présence d'une base telle que la triéthylamine, et à le faire réagir avec le composé de formule (II), dans un solvant tel que le dichlorométhane, sous une atmosphère inerte, à la température ambiante, en présence d'une base telle que la triéthylamine.

Lorsqu'on traite un composé de formule (II) avec un halogénure de sulfonyle de formule (IV), on opère en présence d'une base telle que la triéthylamine ou la diisopropyléthylamine, dans un solvant tel que le dichlorométhane ou le tétrahydrofurane et à une température comprise entre la température ambiante et la température de reflux du solvant.

Lorsqu'on traite un composé de formule (II) avec un halogénoformiate de formule (V), on opère en présence d'une base telle que la triéthylamine, dans un solvant tel que le dichlorométhane et à une température comprise entre 0°C et la température ambiante. Puis on fait réagir le composé intermédiaire de formule (VI) ainsi obtenu avec une amine de formule (VII), dans un solvant tel que le dichlorométhane, en présence d'une base telle que la triéthylamine et à une température comprise entre 0°C et la température de reflux du solvant.

Selon une variante du procédé on peut préparer les composés de formule (I) dans laquelle -X- représente un groupe -CON(R₆)- dans lequel R₆ = H par réaction d'un composé de formule (II) avec un isocyanate de formule R₁-N=C=O (VIII), dans un solvant tel que le dichlorométhane et à une température comprise entre la température ambiante et la température de reflux du solvant.

Selon une autre variante du procédé on peut préparer les composés de formule (I) dans laquelle -X- représente un groupe -CON(R₆)- par réaction d'un composé de formule (II) avec un composé de formule ClCON(R₆)R₁ (IX) en présence d'une base telle que la triéthylamine, dans un solvant tel que le dichlorométhane et à une température comprise entre 0°C et la température ambiante.

Selon une autre variante du procédé on peut préparer un composé de formule (I) dans laquelle R₂ représente un (C₁-C₃)alkyle par réaction d'un composé de formule (I) dans laquelle R₂ = H avec un halogénure de (C₁-C₃)alkyle, en présence d'une base telle que l'hydrure de sodium, dans un solvant tel que le N,N-diméthylformamide et à une température comprise entre la température ambiante et la température de reflux du solvant.

Les composés de formule (I) ainsi obtenus peuvent être ultérieurement séparés du milieu réactionnel et purifiés selon les méthodes classiques, par exemple par cristallisation ou chromatographie.

Les composés de formule (II) se préparent par réaction d'un composé de formule : 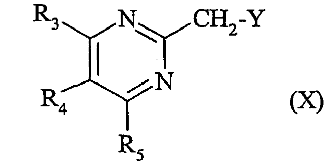 dans laquelle R₃, R₄, R₅ sont tels que définis pour un composé de formule (I) et Y représente un groupe partant tel que défini ci-dessus, de préférence un atome d'halogène ou un groupe hydroxy activé tel qu'un groupe méthanesulfonate, benzène sulfonate, p-toluènesulfonate ou triflate, avec un composé de formule :

H₂N-R₂ (XI)

dans laquelle R₂ est tel que défini pour un composé de formule (I).

La réaction s'effectue dans un solvant tel que le N,N-diméthylformamide, l'acétonitrile, le dichlorométhane, le toluène ou le propan-2-ol, et en présence ou en l'absence d'une base. Lorsqu'on utilise une base, celle-ci est choisie parmi les bases organiques telles que la triéthylamine, la N,N-diisopropyléthylamine ou la N-méthylmorpholine. La réaction s'effectue à une température comprise entre 0°C et la température de reflux du solvant.

Selon une variante, on peut également préparer un composé de formule (II) dans laquelle R₂ = H par réaction d'un composé de formule (X) dans laquelle Y = C1 avec le 1,3,5,7-tétraazatricyclo[3.3.1^{3,7}]décane (ou hexaméthylènetétramine) en présence d'un halogénure de métal alcalin tel que l'iodure de sodium, dans un solvant tel que l'éthanol, à une température comprise entre la température ambiante et la température de reflux du solvant, suivi d'une hydrolyse par un acide fort tel que l'acide chlorhydrique.

Selon une autre variante, on peut également préparer un composé de formule (II) dans laquelle R₂ = H par réduction d'un composé de formule : 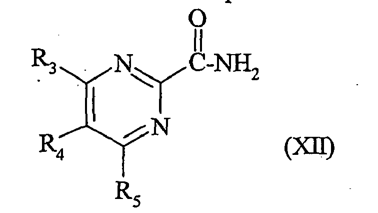 dans laquelle R₃, R₄ et R₅ sont tels que définis pour un composé de formule (I). La réduction s'effectue au moyen d'un agent réducteur tel que le borane dans un solvant tel que le tétrahydrofurane, à une température comprise entre la température ambiante et la température de reflux du solvant, suivi d'une hydrolyse acide.

Selon une autre variante enfin, on peut également préparer un composé de formule (II) dans laquelle R₂ = H par réaction d'un composé de formule : 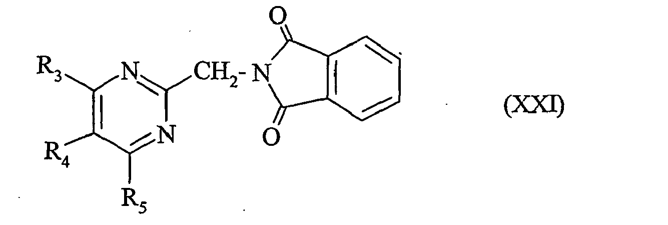 dans laquelle R₃, R₄ et R₅ sont tels que définis pour un composé de formule (I), avec l'hydrate d'hydrazine, dans un solvant tel que le méthanol et à une température comprise entre la température ambiante et la température de reflux du solvant.

Les composés de formule (III) sont connus.

Les composés de formule (IV) sont disponibles dans le commerce ou décrits dans la littérature, ou peuvent être préparés selon des méthodes qui y sont décrites telles que dans J. Org. Chem. USSR, 1970, 6, 2454-2458 ; J. Am. Chem. Soc., 1952, 74, 2008 ; J. Med.Chem., 1977, 20(10), 1235-1239 ; EP0469 984 ; WO95/18105.

Par exemple les composés de formule (IV) peuvent être préparés par halogénation des acides sulfoniques correspondants ou de leurs sels, par exemple de leur sels de sodium ou de potassium. La réaction s'effectue en présence d'un agent halogénant tel que l'oxychlorure de phosphore, le chlorure de thionyle, le trichlorure de phosphore, le tribromure de phosphore ou le pentachlorure de phosphore, sans solvant ou dans un solvant tel qu'un hydrocarbure halogéné ou le N,N-diméthylformamide et à une température comprise entre -10°C et 200°C.

Les composés de formule (V), (VII), (VIII) et (IX) sont connus ou se préparent selon des méthodes connues.

Les composés de formule (X) se préparent à partir des composés de formule : 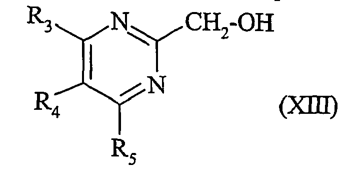 dans laquelle R₃, R₄, R₅ sont tels que définis pour un composé de formule (I), selon des méthodes classiques précédemment citées.

Ainsi, par exemple, lorsque dans un composé de formule (X), Y représente un atome d'halogène, on traite un composé de formule (XIII) par un agent d'halogénation tel que PCl₅, PBr₃, HBr ou BBr₃, dans un solvant tel que le dichlorométhane et à une température comprise entre 0°C et la température ambiante.

Lorsque, dans un composé de formule (X), Y représente un méthanesulfonate, un benzènesulfonate, un p-toluènesulfonate ou un trifluorométhanesulfonate, on fait réagir un composé de formule (XIII) avec un chlorure de sulfonyle de formule W-SO₂-Cl dans laquelle W représente un méthyle, un phényle, un p-tolyle ou un trifluorométhyle. La réaction s'effectue en présence d'une base telle que la triéthylamine, la pyridine ou la N,N-diisopropyléthylamine, dans un solvant tel que le dichlorométhane ou le toluène et à une température comprise entre -20°C et la température de reflux du solvant.

Les composés de formule (X) dans laquelle R₃ = H peuvent également se préparer par réaction d'un composé de formule : 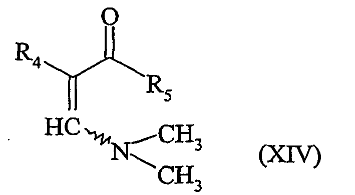 dans laquelle R₄ et R₅ sont tels que définis pour un composé de formule (I) avec le chlorhydrate de chloroacétamidine, dans un solvant tel que l'EtOH ou le MeOH, en présence d'une base telle que le méthylate de sodium, et à une température comprise entre 0°C et la température ambiante.

Les composés de formule (XI) sont connus.

Les composés de formule (XII) se préparent par réaction d'un acide ou d'un dérivé fonctionnel de cet acide de formule : 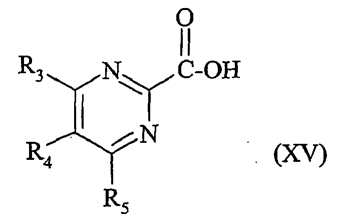 dans laquelle R₃, R₄ et R₅ sont tels que définis pour un composé de formule (I), avec l'ammoniaque selon les méthodes précédemment citées pour la réaction d'un composé (II) avec un composé (III).

Les composés de formule (XIII) se préparent selon les méthodes décrites dans WO 2004/110453 et telles qu'illustrées dans le schéma réactionnel ci-après.

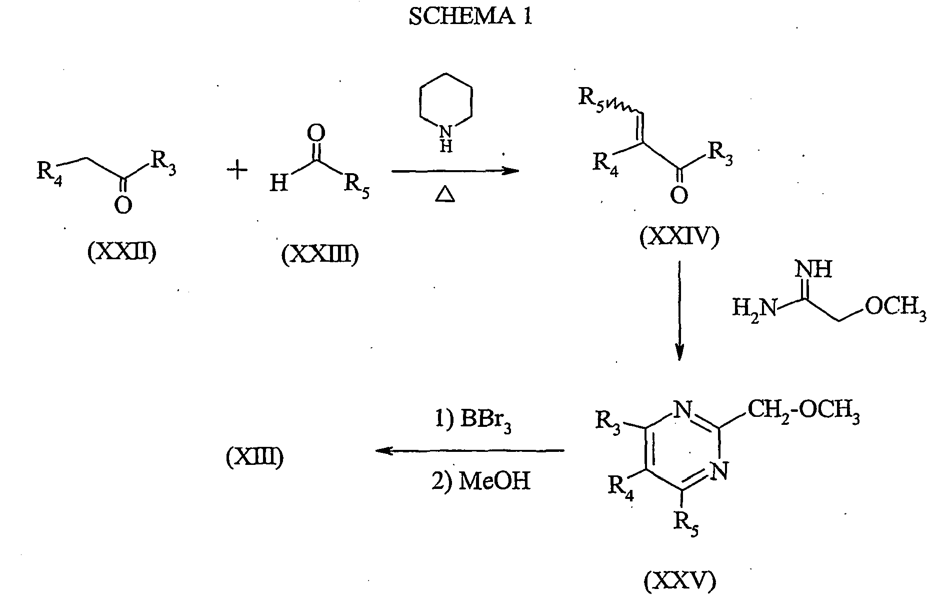

Les composés de formule (XIII) peuvent également se préparer par réduction des composés de formule : 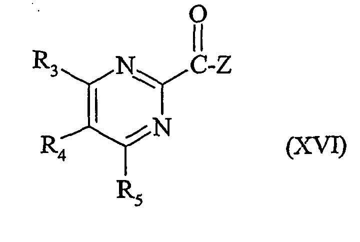 dans laquelle R₃, R₄, R₅ sont tels que définis pour un composé de formule (I) et Z représente un (C₁-C₂)alcoxy.

La réaction s'effectue en présence d'un agent réducteur tel que le borohydrure de sodium ou l'hydrure d'aluminium et de lithium, dans un solvant tel que le tétrahydrofurane, et à une température comprise entre -20°C et la température ambiante.

Les composés de formule (XIV) se préparent par réaction d'un composé de formule : 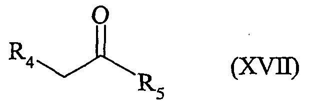 dans laquelle R₄ et R₅ sont tels que définis pour un composé de formule (I) avec le N,N-diméthylformamide diméthylacétal, dans un solvant tel que le tétrahydrofurane et à une température comprise entre la température ambiante et la température de reflux du solvant.

Les composés de formule (XV) se préparent selon les méthodes décrites dans WO 2004/110453.

Les composés de formule (XV) dans laquelle R₃ = H peuvent se préparer par oxydation des composés de formule : 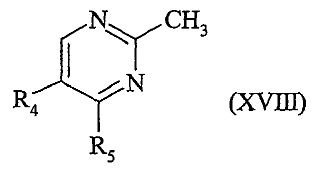 dans laquelle R₄ et R₅ sont tels que définis pour un composé de formule (I) en présence d'un agent oxydant tel que le dioxyde de sélénium, dans un solvant tel que la pyridine et à une température comprise entre la température ambiante et la température de reflux du solvant.

Les composés de formule (XVI) se préparent par réaction d'estérification des composés de formule (XV) selon les méthodes classiques.

Les composés de formule (XVII) se préparent par réaction d'un composé de formule R₄-CH₂-COOH (XIX) avec un composé de formule R₅-COOMe (XX), en présence d'un sel de métal alcalin de l'hexaméthyldisilazane tel que le sel de sodium par exemple, dans un solvant tel que le tétrahydrofurane et à une température comprise entre -70°C et 0°C.

Les composés se formule (XVIII) se préparent par réaction d'un composé de formule (XIV) avec le chlorhydrate d'acétamidine, en présence d'une base telle que le méthylate de sodium, dans un solvant tel que l'éthanol ou le méthanol et à une température comprise entre la température ambiante et la température de reflux du solvant.

Les composés de formule (XIX) et (XX) sont connus ou préparés selon des méthodes connues.

Les composés de formule (XXI) se préparent par réaction d'un composé de formule (XIII) avec le phtalimide, en présence de triphénylphosphine et de diéthylazo dicarboxylate, dans un solvant tel que le tétrahydrofurane et à une température comprise entre -20°C et la température ambiante.

Les composés de formule (XXII) et (XXIII) sont connus ou préparés selon des méthodes connues.

Les EXEMPLES suivants décrivent la préparation de certains composés conformes à l'invention. Ces exemples ne sont pas limitatifs et ne font qu'illustrer la présente invention. Les numéros des composés exemplifiés renvoient à ceux donnés dans le TABLEAU X ci-après, qui illustre les structures chimiques et les propriétés physiques de quelques composés selon l'invention.

Dans les Préparations et dans les Exemples on utilise les abréviations suivantes :
éther : éther diéthylique
éther iso : éther diisopropylique
DMSO : diméthylsulfoxyde
DMF : N,N-diméthylformamide
THF : tétrahydrofurane
PyBOP : hexafluorophosphate de benzotriazol-1-yloxytris(pyrrolidino) phosphonium.
TBTU : tétrafluoroborate de 2-(*1H*-benzotriazol-1-yl)-1,1,3,3-tétraméthyluronium
DCM : dichlorométhane
AcOEt : acétate d'éthyle
DIPEA : diisopropyléthylamine
DBU : 1,8-diazabicyclo[5,4,0]undec-7-ène
TFA : acide trifluoroacétique
Ether chlorhydrique 2N : solution 2N d'acide chlorhydrique dans l'éther diéthylique
F : point de fusion
TA : température ambiante
Eb : température d'ébullition
CLHP : chromatographie liquide haute performance
Silice H : gel de silice 60 H commercialisé par Merck (DARMSTAD)

Solution tampon pH = 2 : solution de 16,66 g de KHSO₄ et 32,32 g de K₂SO₄ dans 1 litre d'eau.

Les spectres de résonance magnétique nucléaire du proton (RMN ¹H) sont enregistrés à 200 MHz dans le DMSO-d₆. Les déplacements chimiques δ sont exprimés en parties par million (ppm). Pour l'interprétation des spectres, on utilise les abréviations suivantes : s : singulet, d : doublet, t : triplet, q : quadruplet, m : massif, mt : multiplet, se : singulet élargi, dd : doublet dédoublé.

Les composés selon l'invention sont analysés par couplage LC/UV/MS (chromatographie liquide/détection UV/spectrométrie de masse). On mesure le pic moléculaire (MH⁺) et le temps de rétention (tr) en minutes.

### Méthode 1 :

On utilise une colonne Symmetry C18 de 2,1 x 50 mm, 3,5 µm, à 30°C, débit 0,4 ml/minute.

L'éluant est composé comme suit :
- solvant A : 0,005 % d'acide trifluoroacétique (TFA) dans l'eau à pH 3,15 ;
- solvant B : 0,005 % de TFA dans l'acétonitrile.

### Gradient :

| Temps (mn) | % A | % B |
|---|---|---|
| 0 | 100 | 0 |
| 10 | 10 | 90 |
| 15 | 10 | 90 |
| 16 | 100 | 0 |
| 20 | 100 | 0 |

La détection UV est effectuée à λ = 210 nM et la détection de masse en mode ionisation chimique ESI positif afin d'observer les ions issus de la protonation des composés analysés (MH⁺).

### Méthode 2 :

On utilise une colonne XTerra MS C18 de 2,1 x 30 mm, 3,5 µm, à 30°C, débit 0,8 ml/minute.

L'éluant est composé comme suit :
- solvant A : 0,025 % de TFA dans l'eau ;
- solvant B : 0,025 % de TFA dans l'acétonitrile.

### Gradient :

| Temps (mn) | % A | % B |
|---|---|---|
| 0 | 100 | 0 |
| 2 | 0 | 100 |
| 2,7 | 0 | 100 |
| 2,75 | 100 | 0 |

La détection UV est effectuée par un détecteur à barette de diodes entre 210 et 400 nm et la détection de masse en mode ionisation chimique ESI positif.

### PREPARATIONS

1. Préparations des composés de formule (XVII)

### Préparation 1.1

2-(4-Bromophényl)-1-(2,4-dichlorophényl)éthanone.

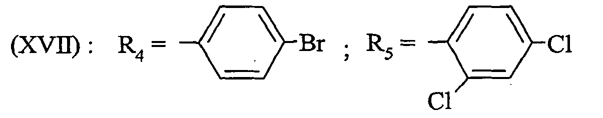

On refroidit à -60°C, sous atmosphère d'azote, 436 ml d'une solution 2M du sel de sodium de l'hexaméthyldisilazane dans le THF, ajoute 400 ml de THF puis, goutte à goutte, une solution de 75 g d'acide 4-bromophénylacétique dans 100 ml de THF et laisse 1 heure 30 minutes sous agitation à -70°C. On ajoute ensuite, goutte à goutte, 67,9 g de 2,4-dichlorobenzoate de méthyle, laisse 30 minutes sous agitation puis laisse remonter la température à 5°C. On verse le mélange réactionnel sur un mélange glace/1 litre d'HCl 2N, extrait à l'éther, lave la phase organique par une solution saturée de NaHCO₃, à l'eau, sèche sur Na₂SO₄ et évapore le solvant sous vide jusqu'à un volume de 200 ml, ajoute du pentane et essore le produit cristallisé formé. On obtient 80 g du composé attendu.

En suivant le mode opératoire décrit à la Préparation 1.1 on prépare les composés de formule (XVII) rassemblés dans le TABLEAU I ci-après :

**TABLEAU I**

| 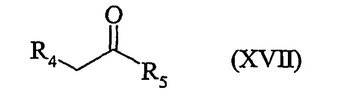 | | |
|---|---|---|
| Préparations | R₄ | R₅ |
| 1.2 | 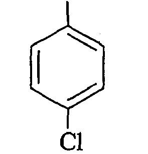 | 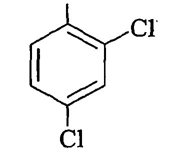 |
| 1.3 | 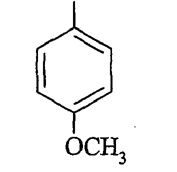 | 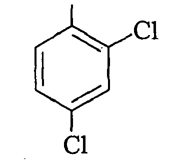 |

### 2. Préparations des composés de formule (XIV).

### Préparation 2.1

### 2-(4-Bromophényl)-1-(2,4-dichlorophényl)-3-(diméthylamino)prop-2-en-1-one.

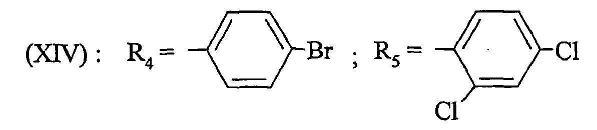

On chauffe à reflux pendant 3 heures un mélange de 30 g du composé de la Préparation 1.1 et 31,2 g de N,N-diméthylformamide diméthylacétal dans 100 ml de THF. On concentre le mélange réactionnel sous vide, reprend le résidu dans l'éther iso, laisse sous agitation, essore le produit cristallisé formé et le lave au pentane. On obtient 32,98 g du composé attendu.

En suivant le mode opératoire décrit à la Préparation 2.1 on prépare les composés de formule (XIV) rassemblés dans le TABLEAU II ci-après :

**TABLEAU II**

| 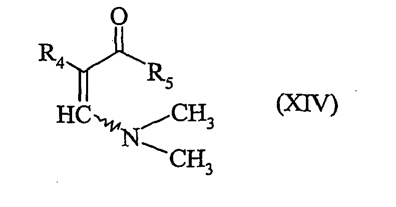 | | |
|---|---|---|
| Préparations | R₄ | R₅ |
| 2.2 | 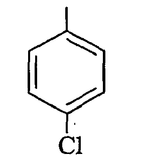 | 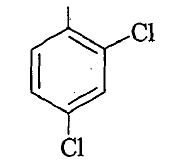 |
| 2.3 | 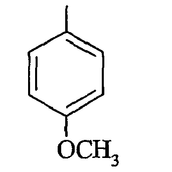 | 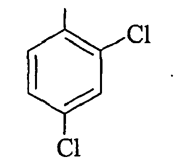 |

### 3. Préparations des composés de formule (X).

### Préparation 3.1

### 5-(4-Bromophényl)-2-(chlorométhyl)-4-(2,4-dichlorophényl)pyrimidine.

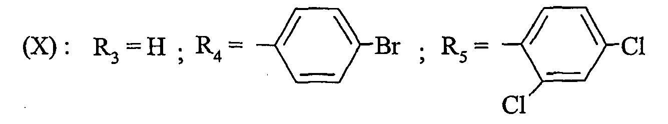

On laisse 10 minutes sous agitation à TA un mélange de 17,6 g du composé de la Préparation 2.1 et 8,5 g de chlorhydrate de chloroacétamidine dans 100 ml d'EtOH, puis ajoute 12,5 ml d'une solution à 30 % de MeONa dans le MeOH et laisse 2 heures sous agitation à TA. On rajoute ensuite 8,5 g de chlorhydrate de chloroacétamidine, laisse 10 minutes sous agitation à TA puis ajoute 12,5 ml d'une solution à 30 % de MeONa dans le MeOH et laisse une nuit sous agitation à TA. On verse le mélange réactionnel dans 500 ml d'un mélange eau/glace, extrait à l'éther, lave la phase organique par une solution saturée de NaCl, sèche sur MgSO₄ et évapore le solvant sous vide. On chromatographie le résidu sur gel de silice en éluant à l'heptane puis par le gradient du mélange heptane/AcOEt de (99/1 ; v/v) jusqu'à (80/20 ; v/v). On obtient 11,9 g du composé attendu.

En suivant le mode opératoire décrit à la Préparation 3.1 on prépare les composés de formule (X) rassemblés dans le TABLEAU III ci-après :

**TABLEAU III**

| | | |
|---|---|---|
| 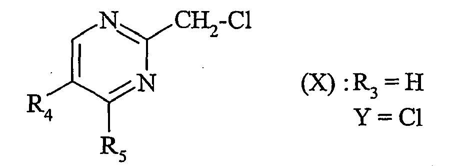 | | |

| Préparations | R₄ | R₅ |
|---|---|---|
| 3.2 | 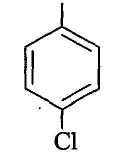 | 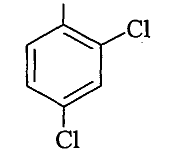 |
| 3.3 | 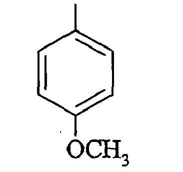 | 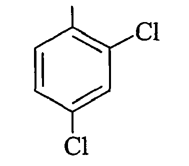 |

### 4. Préparations des composés de formule (II)

### Préparation 4.1

### Chlorhydrate de 1-[5-(4-bromophényl)-4-(2,4-dichlorophényl)pyrimidin-2-yl] méthanamine.

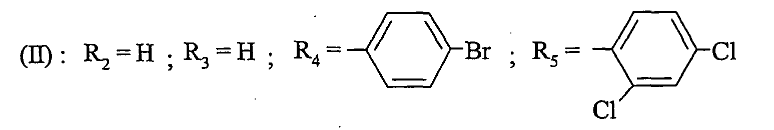

On laisse une nuit sous agitation à TA un mélange de 11 g du composé de la Préparation 3.1, 4,35 g d'hexaméthylènetétramine et 4,03 g d'iodure de sodium dans 220 ml d'EtOH puis chauffe à 50°C pendant 5 heures. On ajoute ensuite 27 ml d'une solution concentrée d'HCl et chauffe à 80°C pendant 5 heures. On concentre sous vide, reprend le résidu dans 2 litres d'eau, lave deux fois la phase aqueuse à l'éther, alcalinise la phase aqueuse par ajout d'une solution concentrée de NaOH, extrait à l'éther, lave la phase organique par une solution saturée de NaCl, sèche sur MgSO₄, filtre, acidifie le filtrat à pH = 1 par ajout d'éther chlorhydrique 2N, essore le précipité formé et le lave à l'éther. On obtient 7,8 g du composé attendu.

En suivant le mode opératoire décrit à la Préparation 4.1 on prépare les composés de formule (II) rassemblés dans le TABLEAU IV ci-après :

**TABLEAU IV**

| | | |
|---|---|---|
| 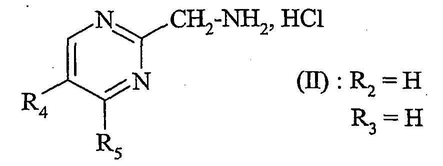 | | |

| Préparations | R₄ | R₅ |
|---|---|---|
| 4.2 | 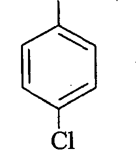 | 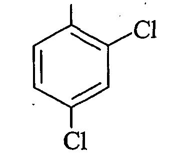 |
| 4.3 | 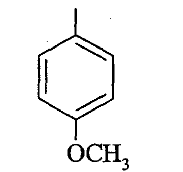 | 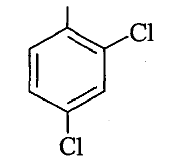 |

### 5. Préparations des composés de formule (XXIV)

### Préparation 5.1

### 3-(4-Bromophényl)-4-(2,4-dichlorophényl)but-3-èn-2-one.

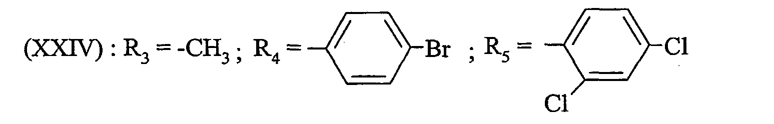

On chauffe à reflux pendant 18 heures un mélange de 15,9 g de 1-(4-bromophényl)acétone, 13 g de 2,4-dichlorobenzaldéhyde de 0,5 g de pipéridine dans 300 ml de benzène, en éliminant l'eau formée à l'aide d'un Dean Stark. On rajoute 0,3 g de pipéridine et poursuit le reflux pendant 24 heures. On concentre le mélange réactionnel sous vide et obtient 27 g du composé attendu sous forme d'huile.

En suivant le mode opératoire décrit à la Préparation 5.1, on prépare les composés de formule (XXIV) rassemblés dans le TABLEAU V ci-après :

**TABLEAU V**

| 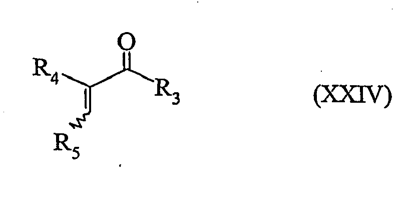 | | | |
|---|---|---|---|
| Préparations | R₃ | R₄ | R₅ |
| 5.2 | -CH₃ | 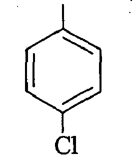 | 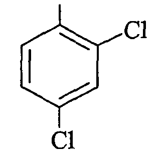 |

### 6. Préparations des composés de formule (XXV)

### Préparation 6.1

### 5-(4-Bromophényl)-4-(2,4-dichlorophényl)-2-(méthoxyméthyl)-6-méthylpyrimidine.

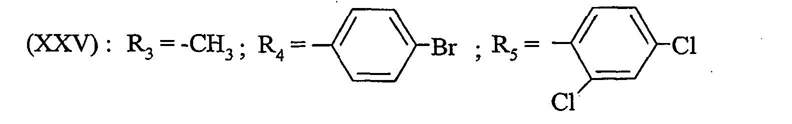

A un mélange de 37 g du composé de la Préparation 5.1 et 25 g de 2-méthoxyéthanimidamide dans 300 ml de DMSO, on ajoute 23 g de *tert*-butylate de potassium, chauffe à 80°C pendant 2 heures puis à 120°C pendant une nuit. On concentre le solvant sous vide jusqu'à un volume de 150 ml, verse le mélange réactionnel sur 2 1 d'eau glacée, extrait à l'éther, lave la phase organique à l'eau, par une solution saturée de NaCl, sèche sur MgSO₄ et évapore le solvant sous vide. On chromatographie le résidu sur gel de silice en éluant à l'heptane puis par un gradient du mélange heptane/AcOEt jusqu'à (50/50 ; v/v). On obtient 6,4 g du composé attendu sous forme de mousse.

En suivant le mode opératoire décrit à la Préparation 6.1 on prépare les composés de formule (XXV) rassemblés dans le TABLEAU VI ci-après :

**TABLEAU VI**

| 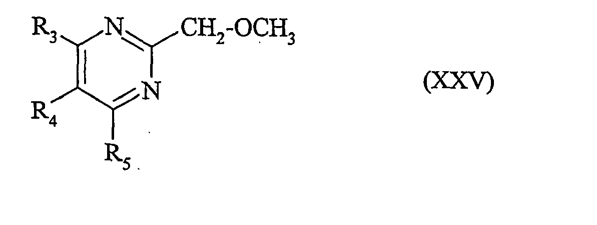 | | | |
|---|---|---|---|
| Préparations | R₃ | R₄ | R₅ |
| 6.2 | -CH₃ | 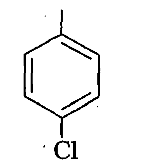 | 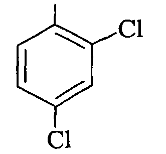 |

### 7. Préparations des composés de formule (XIII)

### Préparation 7.1

### [5-(4-Bromophényl)-4-(2,4-dichlorophényl)-6-méthylpyrimidin-2-yl]méthanol.

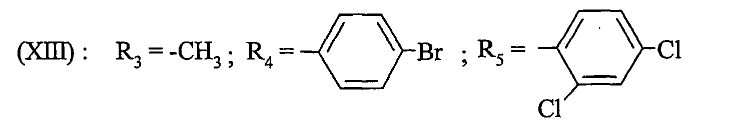

On refroidit à -78°C une solution de 6,4 g du composé de la Préparation 6.1 dans 160 ml de DCM, ajoute goutte à goutte, 24 ml d'une solution 1M de tribromure de bore dans le DCM, laisse sous agitation en laissant remonter la température à 0°C et laisse une heure sous agitation. On refroidit le mélange réactionnel à -78°C, ajoute goutte à goutte 65 ml de MeOH, laisse sous agitation en laissant remonter la température à température ambiante et laisse agiter 2 heures. On concentre le mélange réactionnel sous vide, reprend le résidu au MeOH et évapore sous vide le solvant. On reprend le résidu avec 200 ml de MeOH, ajoute 16 ml d'HCl concentrée et concentre sous vide. On extrait le résidu à l'AcOEt, lave la phase organique à l'eau, par une solution saturée de NaCl, sèche sur MgSO₄ et évapore le solvant sous vide. On obtient 6,1 g du composé attendu.

En suivant le mode opératoire décrit à la Préparation 7.1 on prépare les composés de formule (XIII) rassemblés dans le TABLEAU VII ci-après :

**TABLEAU VII**

| 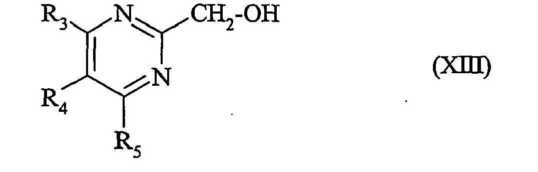 | | | |
|---|---|---|---|
| Préparations | R₃ | R₄ | R₅ |
| 7.2 | -CH₃ | 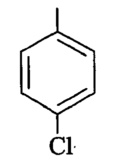 | 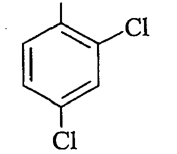 |

### 8. Préparations des composés de formule (XXI)

### Préparation 8.1

### 2-[[5-(4-Bromophényl)-4-(2,4-dichlorophényl)-6-méthylpyrimidin-2-yl]méthyl]-1H-isoindole-1,3-(2H)-dione.

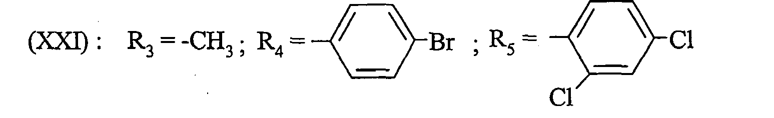

On refroidit à -10°C un mélange de 6,1 g du composé de la Préparation 7.1, 2,33 g de phtalimide et 4,15 g de triphénylphosphine dans 120 ml de THF, ajoute goutte à goutte 2,75 g de diéthylazodicarboxylate, laisse sous agitation en laissant remonter la température à température ambiante et laisse 5 heures sous agitation à TA. On concentre le mélange réactionnel sous vide, extrait le résidu à l'AcOEt, lave la phase organique par une solution tampon pH = 2, à l'eau, par une solution saturée de NaHCO₃ , par une solution saturée de NaCl, sèche sur MgSO₄ et évapore le solvant sous vide. On reprend le résidu à l'éther, laisse sous agitation et essore le produit cristallisé formé. On obtient 3,8 g du composé attendu.

En suivant le mode opératoire décrit à la Préparation 8.1, on prépare les composés de formule (XXI) rassemblés dans le TABLEAU VIII ci-après :

**TABLEAU VIII**

| 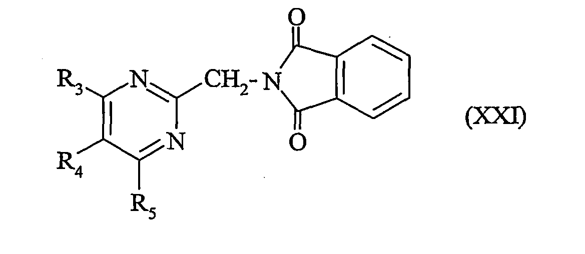 | | | |
|---|---|---|---|
| Préparations | R₃ | R₄ | R₅ |
| 8.2 | -CH₃ | 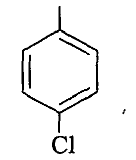 | 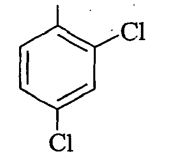 |

### 9. Préparations des composés de formule (II)

### Préparation 9.1

### Chlorhydrate de 1-[5-(4-bromophényl)-4-(2,4-dichlorophényl)-6-méthylpyrimidin-2-yl]méthanamine.

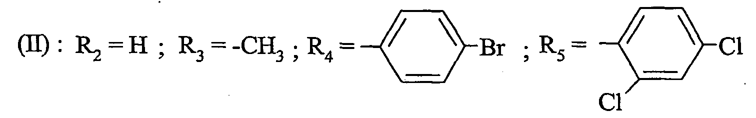

On chauffe à reflux pendant 2 heures 30 minutes un mélange de 3,8 g du composé de la Préparation 8.1 et 0,7 ml d'hydrate d'hydrazine dans 70 ml de MeOH. On concentre le mélange réactionnel sous vide, extrait le résidu à l'éther, lave la phase organique par une solution de NaOH1N, par une solution saturée de NaHCO₃ , par une solution saturée de NaCl, sèche sur MgSO₄ , acidifie jusqu'à pH = 1 par ajout d'éther chlorhydrique 2N, laisse sous agitation et essore le produit cristallisé formé. On obtient 2,9 g du composé attendu.

En suivant le mode opératoire décrit à la Préparation 9.1 on prépare les composés de formule (II) rassemblés dans le TABLEAU IX ci-après :

**TABLEAU IX**

| 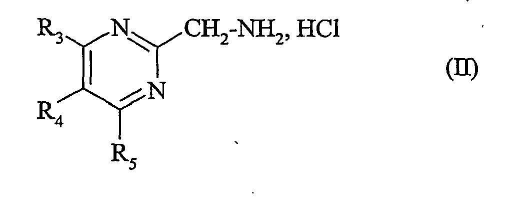 | | | |
|---|---|---|---|
| Préparations | R₃ | R₄ | R₅ |
| 9.2 | -CH₃ | 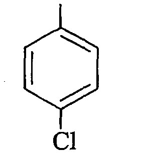 | 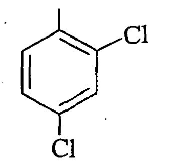 |

### EXEMPLE 1 : Composé N° 1

### N-[[5-(4-Bromophényl)-4-(2,4-dichlorophényl)pyrimidin-2-yl]méthyl]-2-propyl pentanamide.

On laisse une nuit sous agitation à TA un mélange de 0,5 g du composé de la Préparation 4.1, 0,17 g d'acide 2-propylpentanoïque, 0,55 ml de triéthylamine et 0,7 g de PyBOP dans 10 ml de DCM. On concentre sous vide, extrait à l'AcOEt, lave la phase organique par une solution d'HCl 1N, à l'eau, par une solution saturée de NaHCO₃, sèche sur MgSO₄ et évapore le solvant sous vide. On reprend le résidu dans l'EtOH 95, essore le produit cristallisé formé et le lave au pentane. On obtient 0,285 g du composé attendu.

MH⁺ = 534 ; tr = 11,75 (méthode 1).

### EXEMPLE 2 : Composé N° 5

### N-[[5-(4-Bromophényl)-4-(2,4-dichlorophényl)pyrimidin-2-yl]méthyl]-3-chloro benzènesulfonamide.

On laisse 2 heures sous agitation à TA un mélange de 0,5 g du composé de la Préparation 4.1, 0,25 g de chlorure de 3-chlorobenzènesulfonyle et 0,39 ml de triéthylamine dans 10 ml de DCM. On lave le mélange réactionnel par une solution d'HCl 1N, à l'eau, par une solution saturée de NaHCO₃, sèche sur MgSO₄ et évapore le solvant sous vide. On reprend le résidu à l'EtOH et essore le produit cristallisé formé. On obtient 0,44 g du composé attendu.

MH⁺ = 582 ; tr =11,44 (méthode 1).

### EXEMPLE 3 : Composé N° 13

### N-[[5-(4-Chlorophényl)-4-(2,4-dichlorophényl)pyrimidin-2-yl]méthyl] cycloheptanecarboxamide.

On laisse 1 heure sous agitation à TA un mélange de 0,5 g du composé de la Préparation 4.2, 0,18 g d'acide cycloheptanecarboxylique, 0,52 ml de triéthylamine et 0,77 g de PyBOP dans 10 ml de DCM. On lave le mélange réactionnel par une solution d'HCl 1N, à l'eau, par une solution saturée de NaHCO₃, sèche sur sur MgSO₄ et évapore le solvant sous vide. On chromatographie le résidu sur gel de silice en éluant à l'heptane puis par le gradient du mélange heptane/AcOEt de (99/1 ; v/v) jusqu'à (50/50 ; v/v). On obtient 0,39 g du composé attendu.

MH⁺ = 488 ; tr = 11,34 (méthode 1).

### EXEMPLE 4 : Composé N° 33

### N-[[5-(4-Chlorophényl)-4-(2,4-dichlorophényl)pyrimidin-2-yl]méthyl]-N'-[3-(trifluorométhyl)phényl]urée.

On laisse une nuit sous agitation à TA un mélange de 0,5 g du composé de la Préparation 4.2 (base libre) et 0,24 g de 3-(trifluorométhyl)phényl isocyanate dans 5 ml de DCM. On concentre sous vide et chromatographie le résidu sur gel de silice en éluant à l'heptane puis par le gradient du mélange heptane/AcOEt de (99/1 ; v/v) jusqu'à (50/50 ; v/v). On reprend le produit obtenu dans le pentane et essore le précipité formé. On obtient 0,51 g du composé attendu.

MH⁺ = 551 ; tr = 11,54 (méthode 1).

### EXEMPLE 5 : Composé N° 40

### N-[[4-(2,4-dichlorophényl)-5-(4-méthoxyphényl)pyrimidin-2-yl]méthyl]-4-phénoxybenzamide.

On laisse une nuit sous agitation à TA un mélange de 0,4 g du composé de la Préparation 4.3, 0,22 g d'acide 4-phénoxybenzoïque, 0,4 ml de triéthylamine et 0,63 g de PyBOP dans 10 ml de DCM. On concentre sous vide, extrait le résidu à l'éther, lave la phase organique à l'eau, par une solution d'HCl 1N, à l'eau, par une solution saturée de NaHCO₃, sèche sur MgSO₄ et évapore le solvant sous vide jusqu'à un volume résiduel de 5 ml. On laisse sous agitation et essore le produit cristallisé formé. On obtient 0,32 g du composé attendu après cristallisation dans l'EtOH.

MH⁺ = 556 ; tr = 11,19 (méthode 1).

### EXEMPLE 6 : Composé N° 55

### N-[[5-(4-bromophényl)-4-(2,4-dichlorophényl)-6-méthylpyrimidin-2-yl]méthyl]-3,3-diphénylpropanamide.

On laisse une nuit sous agitation à TA un mélange de 0,4 g du composé de la Préparation 9.1, 0,2 g d'acide 3,3-diphénylpropanoïque, 0,3 ml de triéthylamine et 0,55 g de PyBOP dans 10 ml de DCM. On concentre sous vide, extrait le résidu à l'éther, lave la phase organique à l'eau, par une solution d'HCl 1N, à l'eau, par une solution saturée de NaHCO₃ , sèche sur MgSO₄ et évapore le solvant sous vide. On chromatographie le résidu sur gel de silice en éluant à l'heptane puis par le gradient du mélange Heptane/AcOEt jusqu'à (50/50 ; v/v). On obtient 0,3 g du composé attendu après cristallisation dans l'EtOH.

MH⁺ = 630 ; tr = 11,98 (méthode 1).

### EXEMPLE 7 : Composé N° 56

### N-[[5-(4-Bromophényl)-4-(2,4-dichlorophényl)-6-méthylpyrimidin-2-yl]méthyl]-3-(trifluorométhyl)benzènesulfonamide.

A un mélange de 0,4 g du composé de la Préparation 9.1 et 0,3 ml de triéthylamine dans 10 ml de DCM on ajoute 0,24 g de chlorure de 3-(trifluorométhyl)benzènesulfonyle et laisse 15 minutes sous agitation à TA. On lave le mélange réactionnel par une solution d'HCl 1N, à l'eau, par une solution saturée de NaHCO₃, sèche sur MgSO₄ et évapore le solvant sous vide. On reprend le résidu à l'EtOH, laisse sous agitation, essore le produit cristallisé formé et le lave à l'EtOH puis au pentane. On obtient 0,37 g du composé attendu.

MH⁺ = 630 ; tr = 11,96 (méthode 1).

### EXEMPLE 8 : Composé N° 60

### 4-tert-Butyl-N-[[5-(4-chlorophényl)-4-(2,4-dichlorophényl)-6-méthylpyrimidin-2-yl]méthyl]benzamide.

On laisse une nuit sous agitation à TA un mélange de 0,4 g du composé de la Préparation 9.2, 0,18 g d'acide 4-tert-butylbenzoïque, 0,4 ml de triéthylamine et 0,6 g de PyBOP dans 10 ml de DCM. On concentre le mélange réactionnel sous vide, extrait le résidu à l'éther, lave la phase organique à l'eau, par une solution d'HCl 1 N, à l'eau, par une solution saturée de NaHCO₃, sèche sur MgSO₄ et évapore le solvant sous vide. On reprend le résidu dans un minimum d'éther, ajoute de l'éther iso, laisse sous agitation, essore le produit cristallisé formé et le lave à l'éther iso. On obtient 0,35 g du composé attendu.

MH⁺ = 538 ; tr = 12,27 (méthode 1).

Le tableau qui suit illustre les structures chimiques et les propriétés physiques de quelques exemples de composés selon l'invention.

**TABLEAU X**

| | | | | | | | |
|---|---|---|---|---|---|---|---|
| 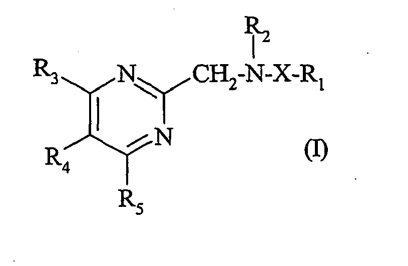 | | | | | | | |

| Composés N° | -X- | R₁ | R₂ | R₃ | R₄ | R₅ | MH⁺; tr (mn) (méthode) solvant de cristallisation |
|---|---|---|---|---|---|---|---|
| 1 | -CO- | -CH(CH₂-CH₂CH₃)₂ | H | H | 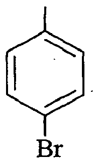 | 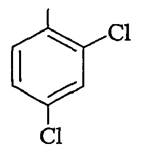 | 534; 11,75 (méthode 1) EtOH 95 |
| 2 | -CO- | 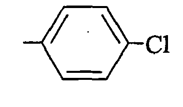 | H | H | 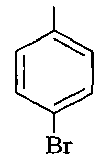 | 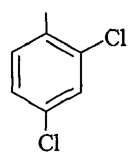 | 546 ; 10,60 (méthode 1) EtOH |
| 3 | -CO- | 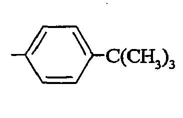 | H | H | 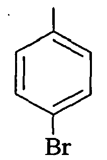 | 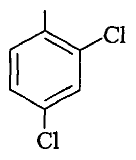 | 568 ; 11,59 (méthode 1) ether iso |
| 4 | -CO- | 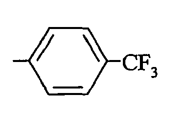 | H | H | 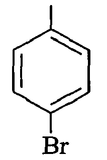 | 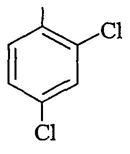 | 580 ; 10,99 (méthode 1) ether iso/pentane |
| 5 | -SO₂- | 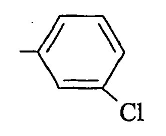 | H | H | 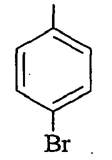 | 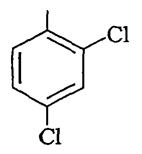 | 582 ; 11,44 (méthode 1) EtOH |
| 6 | -SO₂- | 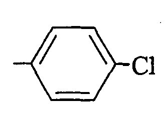 | H | H | 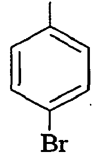 | 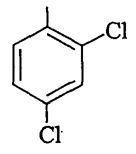 | 582 ; 11,53 (méthode 1) EtOH |
| 7 | -SO₂- | 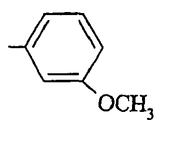 | H | H | 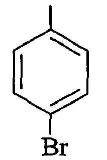 | 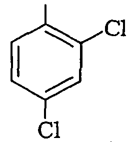 | 578 ; 10,36 (méthode 1) EtOH |
| 8 | -SO₂- | 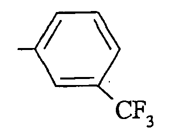 | H | H | 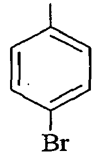 | 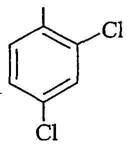 | 616; 11,65 (méthode 1) EtOH RMN |
| 9 | -SO₂- | 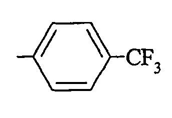 | H | H | 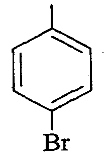 | 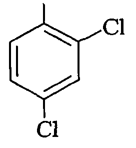 | 616; 11,62 (méthode 1) EtOH RMN |
| 10 | -CO- | -CH(CH₂CH₃)₂ | H | H | 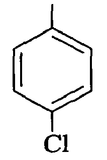 | 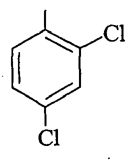 | 462,6 ; 1,91 (méthode 2) |
| 11 | -CO- | -CH(CH₂- CH₂CH₃)₂ | H | H | 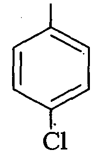 | 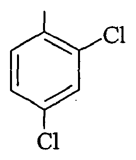 | 490,7 ; 2,02 (méthode 2) |
| 12 | -CO- | 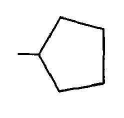 | H | H | 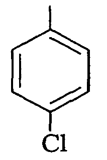 | 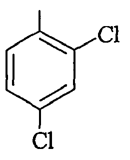 | 460,6 ; 1,89 (méthode 2) |
| 13 | -CO- | 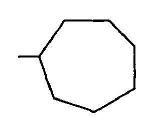 | H | H | 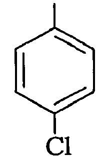 | 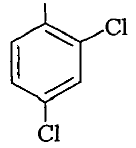 | 488 ; 11,34 (méthode 1) |
| 14 | -CO- | 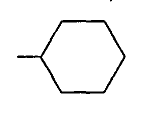 | H | H | 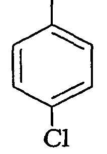 | 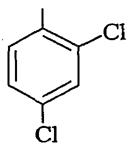 | 474,6; 1,94 (méthode 2) |
| 15 | -CO- | 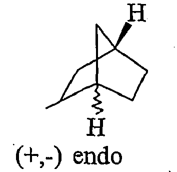 | H | H | 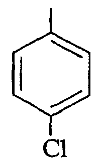 | 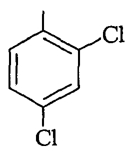 | 486,6 ; 1,97 (méthode 2) |
| 16 | -CO- | 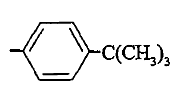 | H | H | 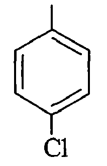 | 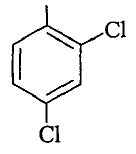 | 524 ; 12,04 (méthode 1) |
| 17 | -CO- | 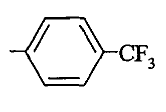 | H | H | 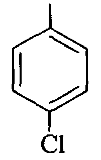 | 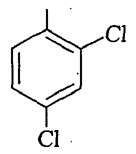 | 536; 11,41 (méthode 1) RMN |
| 18 | -CO- | 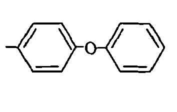 | H | H | 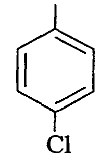 | 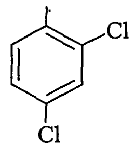 | 560,6 ; 2,04 (méthode 2) |
| 19 | -CO- | 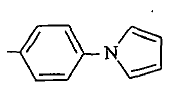 | H | H | 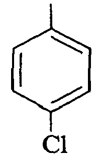 | 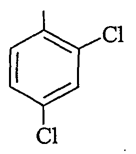 | 533,6 ; 1,99 (méthode 2) |
| 20 | -CO- | 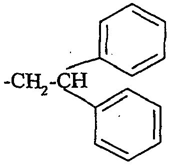 | H | H | 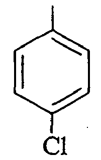 | 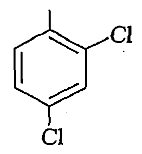 | 572,6 ; 2,01 (méthode 2) |
| 21 | -CO- | 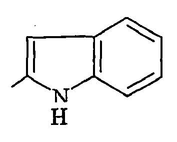 | H | H | 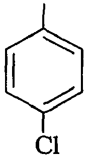 | 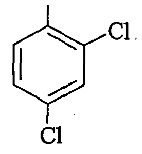 | 507 ; 10,93 (méthode 1) |
| 22 | -CO- | 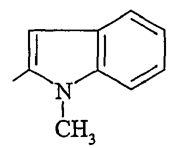 | H | H | 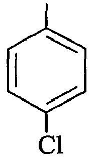 | 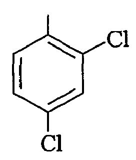 | 521,6 ; 2,01 (méthode 2) |
| 23 | -SO₂- | 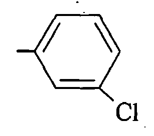 | H | H | 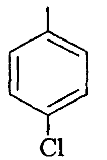 | 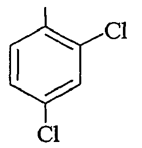 | 538 ; 11,53 (méthode 1) |
| 24 | -SO₂- | 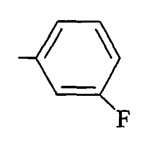 | H | H | 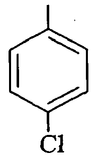 | 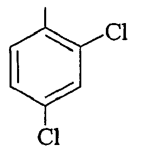 | 521,9; 2,15 (méthode 2) |
| 25 | -SO₂- | 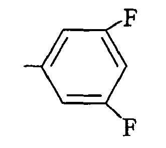 | H | H | 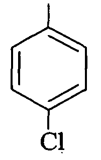 | 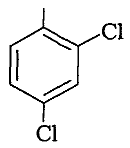 | 539,9 ; 2,18 (méthode 2) |
| 26 | -SO₂- | 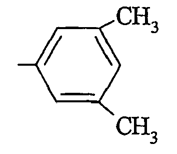 | H | H | 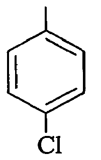 | 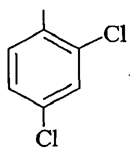 | 531,9 ; 2,22 (méthode 2) |
| 27 | -SO₂- | 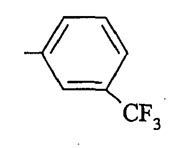 | H | H | 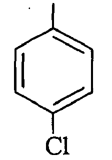 | 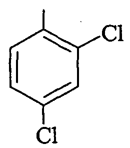 | 571,8 ; 2,21 (méthode 2) |
| 28 | -SO₂- | 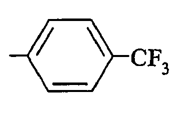 | H | H | 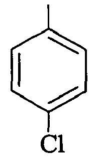 | 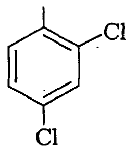 | 572 ; 11,69 (méthode 1) |
| 29 | -SO₂- | 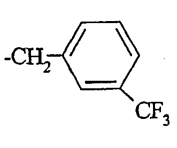 | H | H | 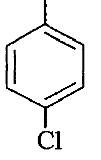 | 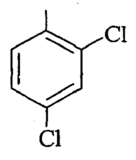 | 586; 11,78 (méthode 1) |
| 30 | -SO₂- | 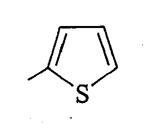 | H | H | 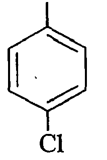 | 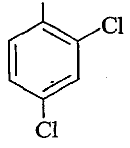 | 509,8 ; 2,12 (méthode 2) |
| 31 | -SO₂- | 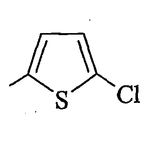 | H | H | 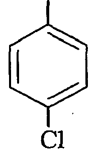 | 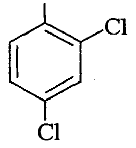 | 543,8 ; 2,22 (méthode 2) |
| 32 | -CONH- | 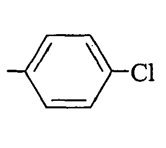 | H | H | 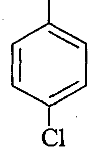 | 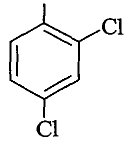 | 516,9; 2,16 (méthode 2) |
| 33 | -CONH- | 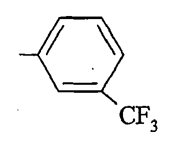 | H | H | 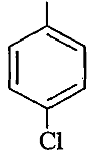 | 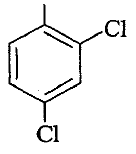 | 551 ; 11,54 (méthode 1) |
| 34 | -CONH- | 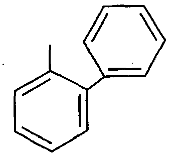 | H | H | 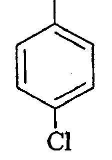 | 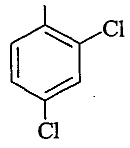 | 559; 11,55 (méthode 1) |
| 35 | -CO- | 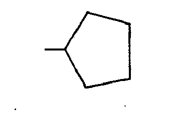 | H | H | 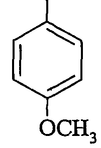 | 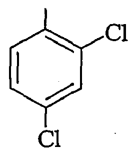 | 456,7 ; 1,80 (méthode 2) |
| 36 | -CO- | 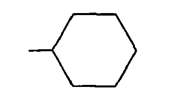 | H | H | 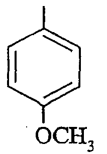 | 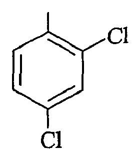 | 470,7 ; 1,85 (méthode 2) |
| 37 | -CO- | 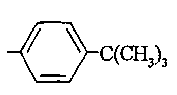 | H | H | 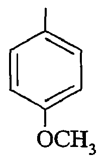 | 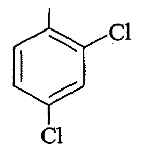 | 520,7 ; 2,01 (méthode 2) |
| 38 | -CO- | 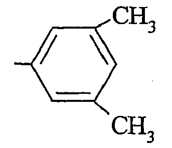 | H | H | 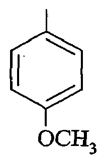 | 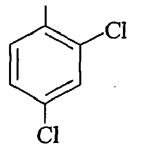 | 492,7 ; 1,91 (méthode 2) |
| 39 | -CO- | 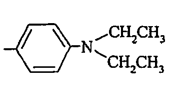 | H | H | 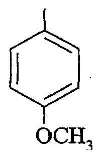 | 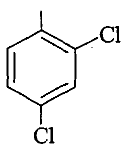 | 535,7 ; 1,71 (méthode 2) |
| 40 | -CO- | 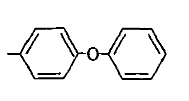 | H | H | 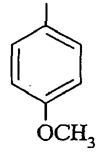 | 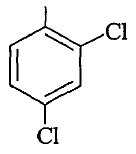 | 556,7 ; 1,96 (méthode 2) |
| 41 | -CO- | 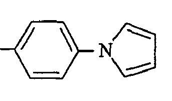 | H | H | 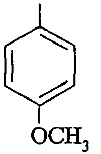 | 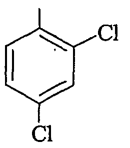 | 529,7 ; 1,91 (méthode 2) |
| 42 | -CO- | 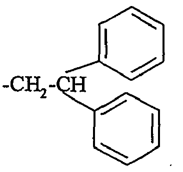 | H | H | 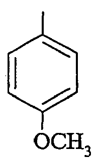 | 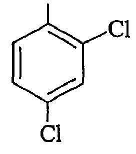 | 568,7 ; 1,93 (méthode 2) |
| 43 | -CO- | 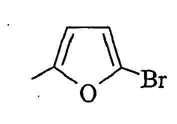 | H | H | 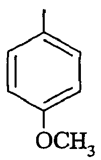 | 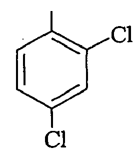 | 532,5 ; 1,85 (méthode 2) |
| 44 | -CO- | 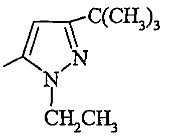 | H | H | 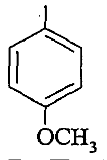 | 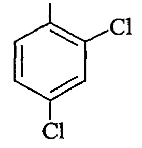 | 538,7 ; 1,94 (méthode 2) |
| 45 | -CO- | 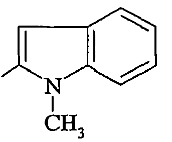 | H | H | 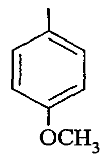 | 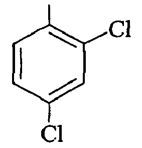 | 517,7 ; 1,93 (méthode 2) |
| 46 | -CONH- | 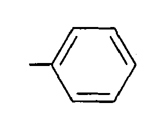 | H | H | 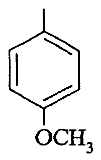 | 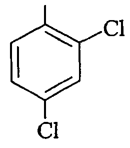 | 479,6 ; 1,79 (méthode 2) |
| 47 | -CONH- | 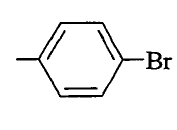 | H | H | 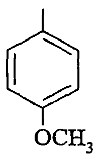 | 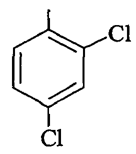 | 557,5 ; 1,90 (méthode 2) |
| 48 | -CONH- | 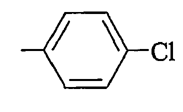 | H | H | 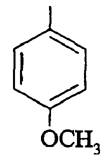 | 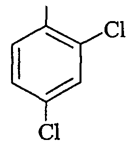 | 513,5 ; 1,88 (méthode 2) |
| 49 | -CONH- | 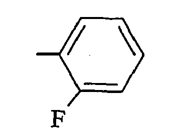 | H | H | 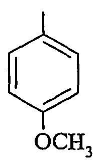 | 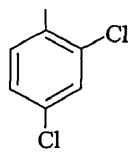 | 497,2 ; 1,82 (méthode 2) |
| 50 | -CONH- | 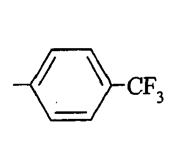 | H | H | 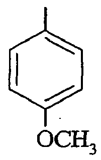 | 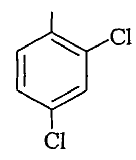 | 547,5 ; 1,92 (méthode 2) |
| 51 | -CO- | 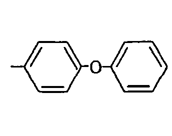 | H | H | 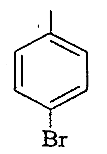 | 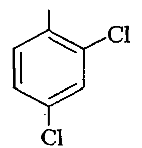 | 604 ; 11,83 (méthode 1) |
| 52 | -CO- | 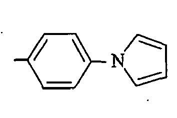 | H | H | 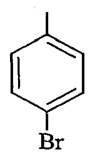 | 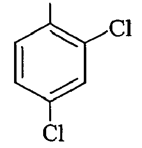 | 576 ; 11,32 (méthode 1) |
| 53 | -CO- | 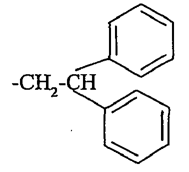 | H | H | 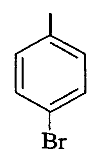 | 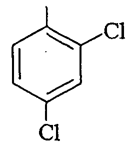 | 616; 11,73 (méthode 1) |
| 54 | -SO₂- | 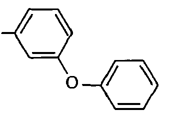 | H | H | 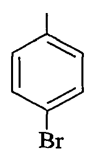 | 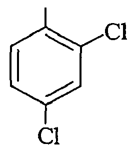 | 640 ; 19,57 (méthode 1) |
| 55 | -CO- | 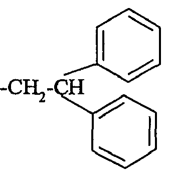 | H | -CH₃ | 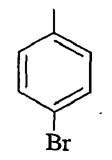 | 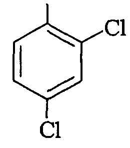 | 630; 11,98 (méthode 1) |
| 56 | -SO₂- | 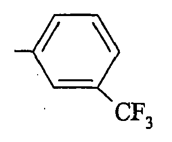 | H | -CH₃ | 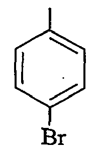 | 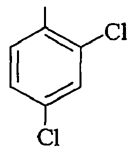 | 630 ; 11,96 (méthode 1) |
| 57 | -SO₂- | 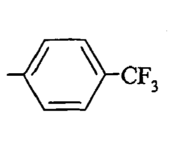 | H | -CH₃ | 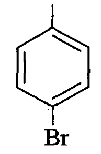 | 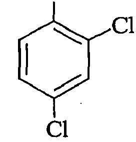 | 630 ; 11,59 (méthode 1) |
| 58 | -SO₂- | 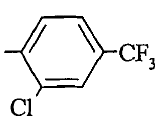 | H | -CH₃ | 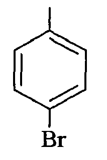 | 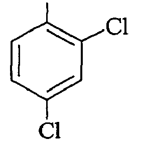 | 664 ; 12,31 (méthode 1) |
| 59 | -SO₂- | 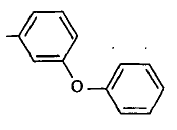 | H | -CH₃ | 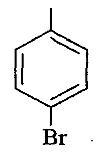 | 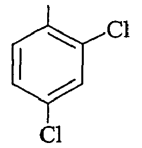 | 654 ; 19,93 (méthode 1) |
| 60 | -CO- | 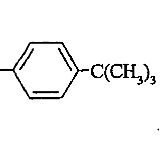 | H | -CH₃ | 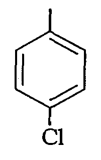 | 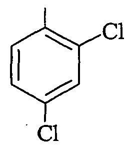 | 538 ; 12,27 (méthode 1) |
| 61 | -CO- | 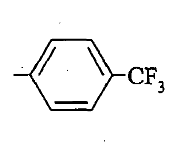 | H | -CH₃ | 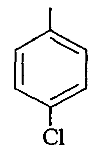 | 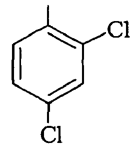 | 550 ; 11,54 (méthode 1) |
| 62 | -SO₂- | 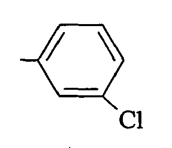 | H | -CH₃ | 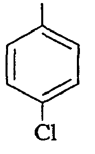 | 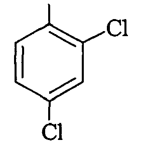 | 552 ; 11,61 (méthode 1) |
| 63 | -SO₂- | 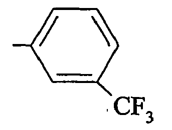 | H | -CH₃ | 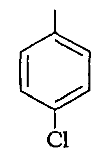 | 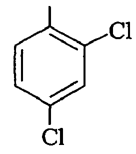 | 586 ; 11,77 (méthode 1) |
| 64 | -SO₂- | 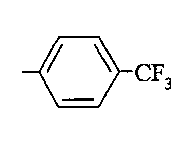 | H | -CH₃ | 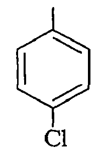 | 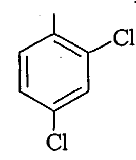 | 586; 11,80 (méthode 1) |
| 65 | -SO₂- | 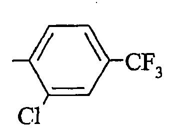 | H | H | 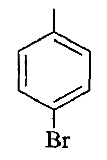 | 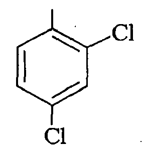 | 650 ; 12,48 (méthode 1) |
| 66 | -SO₂- | 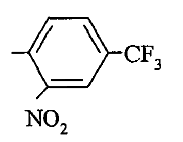 | H | H | 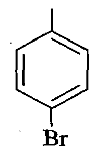 | 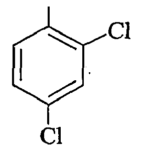 | 661 ; 11,18 (méthode 1) |
| 67 | -SO₂- | 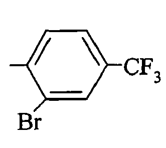 | H | H | 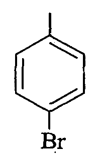 | 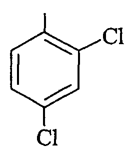 | 694 ; 12,38 (méthode 1) |
| 68 | -SO₂- | 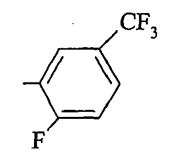 | H | H | 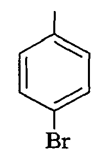 | 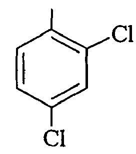 | 634 ; 11,95 (méthode 1) |
| 69 | - SO₂- | 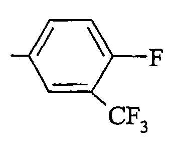 | H | H | 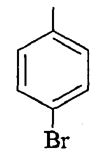 | 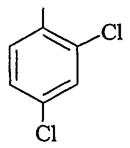 | 634 ; 12,06 (méthode 1) |
| 70 | -SO₂- | 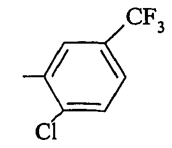 | H | H | 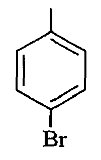 | 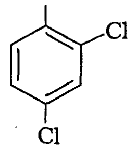 | 650; 12,17 (méthode 1) |
| 71 | -SO₂- | 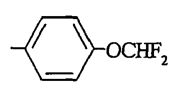 | H | H | 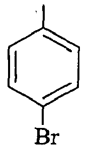 | 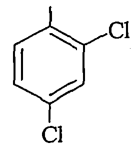 | 614 ; 11,43 (méthode 1) |
| 72 | - SO₂- | 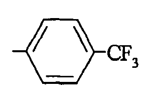 | H | H | 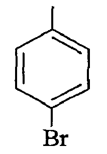 | 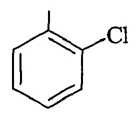 | 582; 11,31 (méthode 1) |

Composé N° 8 : RMN¹H : DMSO-d₆ (200MHz) : δ (ppm) : 4,45 : d : 2H ; 7,05 :
d : 2H ; 7,27-8,13 : m : 9H ; 8,67-8,85 : m : 2H.

Composé N° 9 : RMN¹H : DMSO-d₆ (200MHz) : δ (ppm) : 4,45 : s : 2H ; 7,06 :
d : 2H ; 7,39 : d : 1H ; 7,45-7,63 : m : 4H ; 7,81 : d : 2H ; 7,93 : d : 2H ; 8,73 : s : 1H ; 8,77 : s : 1H.

Composé N° 17 : RMN¹H : DMSO-d₆ (250MHz) : δ (ppm) : 4,77 : d : 2H ; 7,21 :
d : 2H ; 7,40 : d : 2H ; 7,44-7,54 : m : 3H ; 7,87 : d : 2H ; 8,11 : d : 2H ; 8,92 : s : 1H ; 9,41 : t : 1H.

Les composés de formule (I) possèdent une très bonne affinité *in vitro* (IC₅₀ ≤ 5.10⁻⁷M) pour les récepteurs aux cannabinoïdes CB₁, dans les conditions expérimentales décrites par M. Rinaldi-Carmona et al. (FEBS Letters, 1994, 350, 240-244).

La nature antagoniste des composés de formule (I) a été démontrée par les résultats obtenus dans les modèles de l'inhibition de l'adénylate-cyclase comme décrits dans M. Bouaboula et al., J. Biol. Chem., 1995, 270, 13973-13980, M. Rinaldi-Carmona et al., J. Pharmacol. Exp. Ther., 1996, 278, 871-878 et M. Bouaboula et al., J. Biol. Chem., 1997, 272, 22330-22339.

L'interaction d'un composé de formule (I) avec les récepteurs CB₁ présent dans le cerveau est déterminée chez la souris avec le test de binding ex vivo du [3H]-CP55940 après une injection intraveineuse comme décrit dans Rinaldi-Carmona M. et al., FEBS Letters 1994, 350, 240-244 et Rinaldi-Carmona M. et al., Life Sciences 1995, 56, 1941-1947.

L'interaction d'un composé de formule (I) avec les récepteurs CB₁ présent à la périphérie est déterminée chez la souris avec le test de réversion de l'effet inhibiteur du CP55940 sur transit gastrointestinal après une administration orale comme décrit dans Rinaldi-Carmona M. et al., JPET 2004, 310, 905-914.

La toxicité des composés de formule (I) est compatible avec leur utilisation en tant que médicament.

Ainsi, selon un autre de ses aspects, l'invention a pour objet des médicaments pour la médecine humaine ou vétérinaire qui comprennent un composé de formule (I), ou encore un solvat ou un hydrate du composé de formule (I).

Ainsi les composés selon l'invention peuvent être utilisés chez l'homme ou chez l'animal, dans le traitement ou la prévention de maladies impliquant les récepteurs aux cannabinoïdes CB₁, chez l'homme ou chez l'animal notamment chez les mamifères incluant de façon non limitative les chiens, les chats, les chevaux, les bovins, les moutons.

Par exemple et de manière non limitative, les composés de formule (I) sont utiles comme médicaments psychotropes, notamment pour le traitement des désordres psychiatriques incluant l'anxiété, la dépression, les troubles de l'humeur, l'insomnie, les troubles délirants, les troubles obsessionnels, les psychoses en général, la schizophrénie, les troubles de l'attention et de l'hyperactivité (TDAH) chez les enfants hyperkinétiques ainsi que pour le traitement des troubles liés à l'utilisation de substances psychotropes, notamment dans le cas d'un abus d'une substance et/ou de dépendance à une substance, y compris la dépendance alcoolique et la dépendance nicotinique.

Les composés de formule (I) selon l'invention peuvent être utilisés comme médicaments pour le traitement de la migraine, du stress, des maladies d'origine psychosomatique, des crises d'attaques de panique, de l'épilepsie, des troubles du mouvement, en particulier des dyskinésies ou de la maladie de Parkinson, des tremblements et de la dystonie.

Les composés de formule (I) selon l'invention peuvent également être utilisés comme médicaments dans le traitement des troubles mnésiques, des troubles cognitifs, en particulier dans le traitement des démences séniles, de la maladie d'Alzheimer, ainsi que dans le traitement des troubles de l'attention ou de la vigilance. De plus, les composés de formule (I) peuvent être utiles comme neuroprotecteurs, dans le traitement de l'ischémie, des traumatismes crâniens et le traitement des maladies neurodégénératives aigües ou chroniques : incluant la chorée, la chorée de Huntington, le syndrome de Tourrette.

Les composés de formule (I) selon l'invention peuvent être utilisés comme médicaments dans le traitement de la douleur : les douleurs neuropathiques, les douleurs aiguës périphériques, les douleurs chroniques d'origine inflammatoire, les douleurs induites par un traitement anticancéreux.

Les composés de formule (I) selon l'invention peuvent être utilisés comme médicaments dans la médecine humaine ou vétérinaire dans le traitement et la prévention des troubles de l'appétit, de l'appétence (pour les sucres, carbohydrates, drogues, alcools ou toute substance appétissante) et/ou des conduites alimentaires, notamment pour le traitement et la prévention de l'obésité ou de la boulimie ainsi que pour le traitement du diabète de type II ou diabète non insulinodépendant et pour le traitement des dyslipidémies, du syndrome métabolique. Ainsi les composés de formule (I) selon l'invention sont utiles dans le traitement et la prévention de l'obésité et des risques associés à l'obésité, notamment les risques cardio-vasculaires.

De plus, les composés de formule (I) selon l'invention peuvent être utilisés en tant que médicaments dans le traitement et la prévention des troubles gastro-intestinaux, des troubles diarrhéiques, des ulcères, des vomissements, des troubles vésicaux et urinaires, des maladies du foie telles que la cirrhose chronique, la fibrose, la stéatose hépatique, la stéatohépatite, ainsi que des troubles d'origine endocrinienne, des troubles cardio-vasculaires, de l'hypotension de l'arthérosclérose, du choc hémorragique, du choc septique, de l'asthme, de la bronchite chronique, des maladies pulmonaires chroniques obstructives, du syndrome de Raynaud, du glaucome, des troubles de la fertilité, de l'interruption de grossesse, de l'accouchement prématuré, des phénomènes inflammatoires, des maladies du système immunitaire, en particulier autoimmunes et neuroinflammatoires tel que l'arthrite rhumatoïde, l'arthrite réactionnelle, les maladies entraînant une démyélinisation, la sclérose en plaque, des maladies infectieuses et virales telles que les encéphalites, des accidents vasculaires cérébraux ainsi qu'en tant que médicaments pour la chimiothérapie anticancéreuse, pour le traitement du syndrome de Guillain-Barré et pour le traitement des maladies des os et de l'ostéoporose.

Selon la présente invention, les composés de formule (I) sont tout particulièrement utiles pour le traitement des troubles psychotiques, en particulier la schizophrénie, les troubles de l'attention et de l'hyperactivité (TDAH) chez les enfants hyperkinétiques ; pour le traitement des troubles de l'appétit et de l'obésité ; pour le traitement des déficits mnésiques et cognitifs ; pour le traitement de la dépendance alcoolique, de la dépendance nicotinique, c'est à dire pour le sevrage alcoolique et pour le sevrage tabagique ; des maladies neurodégénératives aigües ou chroniques.

Plus particulièrement, les composés de formule (I) selon la présente invention sont utiles dans le traitement et la prévention des troubles de l'appétit, des troubles métaboliques, des troubles gastro-intestinaux, des phénomènes inflammatoires, des maladies du système immunitaire, des troubles psychotiques, de la dépendance alcoolique, de la dépendance nicotinique.

Selon un de ses aspects, la présente invention est relative à l'utilisation d'un composé de formule (I), et de ses solvats ou hydrates pour le traitement des troubles et maladies indiqués ci-dessus.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, un solvat ou hydrate dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Les compositions pharmaceutiques selon la présente invention peuvent contenir à côté d'un composé de formule (I) un (ou plusieurs) autre principe actif utile dans le traitement des troubles et maladies indiquées ci-dessus.

Ainsi, la présente invention a également pour objet des compositions pharmaceutiques contenant un composé de formule (I) selon la présente invention associé à un (ou plusieurs) principe actif choisi parmi l'une des classes thérapeutiques suivantes :
- un autre antagoniste des récepteurs CB₁ aux cannabinoïdes ;
- un modulateur des récepteurs CB₂ aux cannabinoïdes ;
- un antagoniste des récepteurs AT₁ de l'angiotensine II, seul ou associé à un diurétique ;
- un inhibiteur de l'enzyme de conversion, seul ou associé à un diurétique ou à un antagoniste calcique ;
- un antagoniste calcique ;
- un diurétique ;
- un béta-bloquant seul ou associé à un diurétique ou à un antagoniste calcique ;
- un antihyperlipémiant ou un antihypercholestérolémiant ;
- un antidiabétique ;
- un autre agent anti-obésité ou agissant sur les troubles du métabolisme ;
- un agoniste nicotinique, un agoniste nicotinique partiel ;
- un antidépresseur, un antispychotique, un anxiolytique ;
- un anticancéreux ou un agent antiprolifératif ;
- un antagoniste des opioïdes ;
   ainsi que :

- un agent améliorant la mémoire ;
- un agent utile dans le traitement de l'alcoolisme ou des symptômes de sevrage ;
- un agent utile pour traiter l'ostéoporose ;
- un anti-inflammatoire non stéroïdien ou stéroïdien ;
- un anti-infectieux ;
- un analgésique ;
- un antiasthmatique.

Par antagoniste des récepteurs AT₁ de l'angiotensine II, on entend un composé tel que candésartan cilexitil, éprosartan, irbésartan, losartan potassium, olmésartan médoxomil, telmisartan, valsartan, chacun de ces composés pouvant être lui-même associé à un diurétique tel que l'hydrochlorothiazide.

Par inhibiteur de l'enzyme de conversion, on entend un composé tel que alacépril, bénazépril, captopril, cilazapril, énalapril, énalaprilat, fosinopril, imidapril, lisinopril, moexipril, périndopril, quinapril, ramipril, spirapril, temocapril, trandolapril, zofénopril, chacun de ces composés pouvant lui-même être associé à un diurétique tel que l'hydrochlorothiazide ou l'indapamide ou à un antagoniste calcique tel que l'amlodipine, le diltiazem, le félodipine ou le vérapamil.

Par antagoniste calcique, on entend un composé tel que amlodipine, aranidipine, bénidipine, bépridil, cilnidipine, diltiazem, éfonidipine hydrochloride éthanol, fasudil, félodipine, isradipine, lacidipine, lercanidipine hydrochloride, manidipine, mibéfradil hydrochloride, nicardipine, nifédipine, nilvadipine, nimodipine, nisoldipine, nitrendipine, térodiline, vérapamil.

Par béta-bloquant, on entend un composé tel que acébutolol, alprénolol, amosulalol, arotinolol, aténolol, béfunolol, bétaxolol, bévantolol, bisoprolol, bopindolol, bucumolol, bufétolol, bunitrolol, butofilolol, carazolol, cartéolol, carvédilol, cloranolol, épanolol, esmolol, indénolol, labétalol, landiolol, lévobunolol, lévomoprolol, mépindolol, métipranolol, métoprolol, nadolol, nébivolol, nifénalol, nipradilol, oxprénolol, penbutolol, pindolol, propranolol, salmétérol, sotalol, talinolol, tertatolol, tilisolol, timolol, xamotérol, xibénolol.

Par antihyperlipémiant ou antihypercholestérolémiant, on entend un composé choisi parmi les fibrates tels que alufibrate, béclobrate, bézafibrate, ciprofibrate, clinofibrate, clofibrate, étofibrate, fénofibrate ; les statines (inhibiteurs de HMG-CoA reductase), telles que atorvastatine, fluvastatine sodium, lovastatine, pravastatine, rosuvastatine, simvastatine, ou un composé tel que acipimox, aluminum nicotinate, azacostérol, cholestyramine, dextrothyroxine, méglutol, nicéritrol, nicoclonate, acide nicotinique, béta-sitosterol, tiadénol.

Par antidiabétique, on entend un composé appartenant à l'une des classes suivantes : les sulfonylurées, les biguanidines, les inhibiteurs d'alpha glucosidase, les thiazolidinedione, les métiglinides, tel que acarbose, acétohexamide, carbutamide, chlorpropamide, glibenclamide, glibornuride, gliclazide, glimépiride, glipizide, gliquidone, glisoxepide, glybuzole, glymidine, métahexamide, métformin, miglitol, natéglinide, pioglitazone, répaglinide, rosiglitazone, tolazamide, tolbutamide, troglitazone, voglibose, ainsi que l'insuline et les analogues de l'insuline.

Par autre agent anti-obésité ou agissant sur les troubles du métabolisme, on entend un composé tel que amfépramone, benfluorex, benzphétamine, indanorex, mazindole, méfénorex, méthamphétamine, D-norpseudoéphédrine, sibutramine, un inhibiteur de lipase (orlistat cetilistat), un agoniste PPAR (de l'anglais Peroxisome Proliferator Activated Receptor Agonist), un dopamine agoniste, un agoniste des récepteurs de leptine, un inhibiteur du reuptake de la sérotonine, un béta-3 agoniste, un CCK-A agoniste, un inhibiteur de NPY,un agoniste des récepteurs MC4, un antagoniste des récepteurs MCH (de l'anglais Melanin Concentrating Hormone), un antagoniste de l'orexine, un inhibiteur de phosphodiesterase, un inhibiteur de 11βHSD (11-β-hydroxy steroid deshydrogenase), un inhibiteur de DPP-IV (dipeptidyl peptidase IV), un antagoniste (ou agoniste inverse de l'histamine H3, un dérivé CNTF (de l'anglais Ciliary Neurotrophic Factor), un agoniste des récepteurs GHS (de l'anglais Growth Hormone Secretagogue, un modulateur de la ghréline, un inhibiteur de diacyglycerol acyltransferase (DGAT), un inhibiteur de phosphodiesterase (PDE), un agoniste d'hormone thyroïdienne, un antagoniste des récepteurs glucocorticoïdes, un inhibiteur de stearoyl-CoA- desaturase (SCD), un modulateur des transporteurs de phosphate, de glucose, d'acide gras, de dicarboxylate, un antagoniste 5HT₂, un antagoniste 5HT₆, un agoniste de la bombesine.

Par antagoniste des opioïdes on entend un composé tel que naltrexone, naloxone ou nalméfène.

Par agent utile dans le traitement de l'alcoolisme ainsi que des symptômes de sevrage on entend l'acamprosate, les benzodiazepines, les béta-bloquants, la clonidine, la carbamazépine.

Par agent utile, pour traiter l'ostéoporose, on entend par exemple, les biphosphonates tels que étidronate, clodronate, tiludronate, risédronate.

Selon la présente invention, on peut également associer d'autres composés ayant des propriétés antihyperlipemiantes, antihypercholesterolemiantes, antidiabétiques ou anti-obésité. Plus particulièrement on peut associer des composés appartenant à l'une des classes suivantes :
inhibiteurs de PTP 1 B (de l'anglais Protein Tyrosine Phosphase -1B), les agonistes des récepteurs VPAC 2, les modulateurs de GLK, les modulateurs retinoides, les inhibiteurs de glycogen phosporylase (HGLPa), les antagonistes du glucagon, les inhibiteurs de glucose-6 phosphate, les activateurs de pyruvate dehydrogenase kinase (PKD), les modulateurs de RXR, FXR, LXR, les inhibiteurs de SGLT (de l'anglais Sodium Dependant Glucose Transporter), les inhibiteurs de CETP (de l'anglais Cholesterylester Transfer Protein), les inhibiteurs de squalene synthetase, les inhibiteurs de squalene epoxidase, les inhibiteurs de synthèse des triglycérides, les inducteurs de récepteurs LDL (de l'anglais Low Density Lipoprotein), les inhibiteurs de IBAT, les inhibiteurs de FBPase (fructose-1,6-biphosphatase), les modulateurs de CART (de l'anglais Cocaïne-Amphétamine-Regulated Transcript),les modulateurs MC 4 (mélanocortin 4), les antagonistes des récepteurs de l'oréxine.

Selon un autre aspect de l'invention, le composé de formule (I), ou un de ses solvats ou hydrates et l'autre principe actif associé peuvent être administrés de manière simultanée, séparée ou étalée dans le temps.

On entend par « utilisation simultanée » l'administration des composés de la composition selon l'invention compris dans une seule et même forme pharmaceutique.

On entend par « utilisation séparée » l'administration, en même temps, des deux composés de la composition selon l'invention chacun compris dans une forme pharmaceutique distincte.

On entend par « utilisation étalée dans le temps » l'administration successive, du premier composé de la composition de l'invention, compris dans une forme pharmaceutique, puis du deuxième composé de la composition selon l'invention, compris dans une forme pharmaceutique distincte. Dans ce cas, le laps de temps écoulé entre l'administration du premier composé de la composition selon l'invention et l'administration du deuxième composé de la même composition selon l'invention n'excède généralement pas 24 heures.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention : | 50,0 mg |
| Mannitol : | 223,75 mg |
| Croscarmellose sodique : | 6,0 mg |
| Amidon de maïs : | 15,0 mg |
| Hydroxypropyl-méthylcellulose : | 2,25 mg |
| Stéarate de magnésium : | 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou hydrates ou solvats.

## Revendications

1. Composé répondant à la formule (I) : 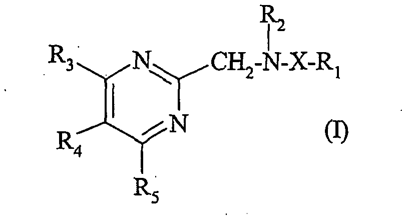 dans laquelle :
- X représente un groupe 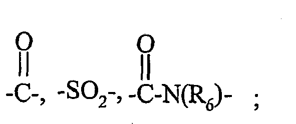
- R₁ représente :
. un (C₁-C₁₂)alkyle non substitué ou substitué une ou plusieurs fois par des substituants choisis indépendamment parmi un atome de fluor, un hydroxyle, un (C₁-C₄) alcoxy, un (C₁-C₄)alkylthio, un phénoxy, un radical trifluorométhoxy, un radical difluorométhoxy, un radical difluorométhylthio, un radical trifluorométhylthio ;
. un radical carbocyclique non aromatique en (C₃-C₁₂) non substitué ou substitué une ou plusieurs fois par des substituants choisis indépendamment parmi un (C₁-C₄)alkyle, un (C₁-C₄)alcoxy, un atome de fluor, un hydroxyle, un radical trifluorométhyle, un radical trifluorométhoxy, un (C₁-C₄)alkylthio ;
. un (C₃-C₇)cycloalkylméthyle non substitué ou substitué une ou plusieurs fois sur le carbocycle par des substituants choisis indépendamment parmi un (C₁-C₄)alkyle, un (C₁-C₄)alcoxy, un atome de fluor, un hydroxyle, un radical trifluorométhyle, un radical trifluorométhoxy ;
. un phényle non substitué ou substitué une ou plusieurs fois par des substituants choisis indépendamment parmi un atome d'halogène, un hydroxyle, un groupe Alk, un groupe OAlk, un méthylènedioxy, un (C₁-C₄)alkylamino, un di-(C₁-C₄)alkylamino, un cyano, un nitro, un groupe S(O)ₙAlk, un groupe OS(O)ₙAlk, un groupe (C₁-C₄)alkylcarbonyle ; ou parmi un radical phényle, phénoxy, pyrrolyle, imidazolyle; pyridyle ou pyrazolyle, ledit radical étant non substitué ou substitué une ou plusieurs fois par un (C₁-C₄)alkyle ;
. un benzyle non substitué ou substitué une ou plusieurs fois sur le phényle par des substituants choisis indépendamment parmi un atome d'halogène, un groupe Alk, un hydroxyle, un groupe OAlk, un méthylènedioxy, un groupe S(O)ₙAlk, un groupe OS(O)ₙAlk et non substitué ou substitué en alpha par un ou deux groupes semblables ou différents choisis parmi un (C₁-C₄)alkyle, un (C₃-C₇)cycloalkyle ;
. un phénéthyle non substitué ou substitué une ou plusieurs fois sur le phényle par des substituants choisis indépendamment parmi un atome d'halogène, un (C₁-C₄)alkyle, un (C₁-C₄)alcoxy, un radical trifluorométhyle, un radical trifluorométhoxy ;
. un bemhydryle ; un benzhydrylméthyle ;
. un 1,2,3,4-tétrahydronaphtalènyle non substitué ou substitué une ou plusieurs fois par un (C₁-C₄)alkyle ;
. un radical hétérocyclique aromatique choisi parmi un pyrrolyle, un imidazolyle, un furyle, un thiényle, un pyrazolyle, un oxazolyle, un pyridyle, un indolyle, ledit radical étant non substitué ou substitué une ou plusieurs fois par des substituants choisis indépendamment parmi un atome d'halogène ou un (C₁-C₄)alkyle, un (C₁-C₄)alcoxy, un radical trifluorométhyle, un radical trifluorométhoxy, un cyano, un nitro, un (C₁-C₄)alkylthio ;
- R₂ représente un atome d'hydrogène ou un (C₁-C₃)alkyle ;
- R₃ représente un atome d'hydrogène ou un (C₁-C₅)alkyle ;
- R₄ représente un phényle non substitué ou substitué une ou plusieurs fois par des substituants choisis indépendamment parmi un atome d'halogène, un groupe Alk, un un groupe OAlk, un groupe S(O)ₙAlk ou un groupe OS(O)ₙAlk ;
- R₅ représente un phényle non substitué ou substitué une ou plusieurs fois par des substituants choisis indépendamment parmi un atome d'halogène, un groupe Alk, un groupe OAlk, un groupe S(O)ₙAlk ou un groupe OS(O)ₙAlk ;
- R₆ représente un atome d'hydrogène ou un (C₁-C₃)alkyle ;
- n représente 0, 1 ou 2 ;
- Alk représente un (C₁-C₄)alkyle non substitué ou substitué une ou plusieurs fois par un atome de fluor ;
à la condition que R₄ et R₅ ne représentent pas simultanément un phényle substitué par un (C₁-C₄)alcoxy ; ainsi que leur hydrate ou leur solvat.

2. Composé selon la revendication 1 de formule (IA) dans laquelle -X- représente un radical -CO- et les substituants R₁ à R₅ sont tels que définis pour les composés de formule (I) à la revendication 1 ;
ainsi que ses hydrates ou ses solvats.

3. Composé selon la revendication 1 de formule (IB) dans laquelle -X- représente un radical -SO₂- et les substituants R₁ à R₅ sont tels que définis pour les composés de formule (I) à la revendication 1 ;
ainsi que ses hydrates ou ses solvats.

4. Composé selon la revendication 1 de formule (IC) dans laquelle -X- représente un radical -CON(R₆)- et les substituants R₁ à R₆ sont tels que définis pour les composés de formule (I) à la revendication 1 ;
ainsi que ses hydrates ou ses solvats.

5. Composé de formule (I) selon la revendication 1 dans laquelle :
- X représente un groupe -CO- , un groupe -SO₂- , un groupe -CON(R₆)- ;
- R₁ représente :
. un 1-éthylpropyle ; un 1-propylbutyle ;
. un cyclopentyle ; un cyclohexyle ; un cycloheptyle ; un 2-norbornyle ;
. un phényle ; un 4-bromophényle ; un 3-chlorophényle ; un 4-chlorophényle ; un 2-fluorophényle ; un 3-fluorophényle ; un 3,5-difluorophényle ; un *4-tert-*butylphényle ; un 3,5-diméthylphényle ; un 3-méthoxyphényle ; un 4-(diéthylamino)phényle ; un 3-(trifluorométhyl)phényle ; un 4-(trifluorométhyl) phényle ; un 2-chloro-4-(trifluorométhyl)phényle ; un 2-nitro-4-(trifluorométhyl)phényle ; un 2-bromo-4-(trifluorométhyl)phényle ; un 2-fluoro-5-(trifluorométhyl)phényle ; un 4-fluoro-3-(trifluorométhyl)phényle ; un 2-chloro-5-(trifluorométhyl)phényle ; un 4-(difluorométhoxy)phényle ; un biphényl-2-yle ; un 3-phénoxyphényle ; un 4-phénoxyphényle ; un 4-(1*H*-pyrrol-1-yl) phényle ;
. un 3-(trifluorométhyl)benzyle ;
. un benzhydrylméthyle ;
. un 2-thiényle ; un 5-chloro-2-thiényle ; un 5-bromo-2-furyle ; un 3-*tert*-butyl-1-éthyl-1*H*-pyrazol-5-yle ; un 1*H*-indol-2-yle ; un 1-méthyl-1*H*-indol-2-yle ;
- R₂ représente un atome d'hydrogène ;
- R₃ représente un atome d'hydrogène ou un méthyle ;
- R₄ représente un 4-bromophényle ; un 4-chlorophényle ; un 4-méthoxyphényle ;
- R₅ représente un 2,4-dichlorophényle ; un 2-chlorophényle ;
- R₆ représente un atome d'hydrogène ;
ainsi que leur hydrate ou leur solvat.

6. Composé de formule (I) selon la revendication 1 dans laquelle :
- X représente un groupe -CO- ou -SO₂- ;
- R₁ représente :
. un 3-(trifluorométhyl)phényle ; un 4-(trifluorométhyl)phényle ; un 2-chloro-4-(trifluorométhyl)phényle ; un 3-phénoxyphényle ; un 4-(1H-pyrrol-1-yl)phényle ;
. un benzhydrylméthyle ;
- R₂ représente un atome d'hydrogène ;
- R₃ représente un atome d'hydrogène ou un méthyle ;
- R₄ représente un 4-bromophényle ou un 4-méthoxyphényle ;
- R₅ représente un 2,4-dichlorophényle ; un 2-chlorophényle ;
ainsi que leur hydrate ou leur solvat.

7. Composé de formule (I) selon la revendication 1 choisi parmi :
- N-[[5-(4-bromophényl)-4-(2,4-dichlorophényl)pyrimidin-2-yl]méthyl]-3-(trifluorométhyl)benzènesulfonamide ;
- N-[[5-(4-bromophényl)-4-(2,4-dichlorophényl)pyrimidin-2-yl]méthyl]-4-(trifluorométhyl)benzènesulfonamide ;
- N-[[4-(2,4-dichlorophényl)-5-(4-méthoxyphényl)pyrimidin-2-yl]méthyl]-4-(1*H-*pyrrol-1-yl)benzamide ;
- N-[[5-(4-bromophényl)-4-(2,4-dichlorophényl)pyrimidin-2-yl]méthyl]-3,3-diphénylpropanamide ;
- N-[[5-(4-bromophényl)-4-(2,4-dichlorophényl)-6-méthylpyrimidin-2-yl]méthyl]-3,3-diphénylpropanamide ;
- N-[[5-(4-bromophényl)-4-(2,4-dichlorophényl)-6-méthylpyrimidin-2-yl]méthyl]-3-(trifluorométhyl)benzènesulfonamide ;
- N-[[5-(4-bromophényl)-4-(2,4-dichlorophényl)-6-méthylpyrimidin-2-yl]méthyl]-4-(trifluorométhyl)benzènesulfonamide ;
- N-[[5-(4-bromophényl)-4-(2,4-dichlorophényl)-6-méthylpyrimidin-2-yl]méthyl]-2-chloro-4-(trifluorométhyl)benzènesulfonamide ;
- N-[[5-(4-bromophényl)-4-(2,4-dichlorophényl)-6-méthylpyrimidin-2-yl]méthyl]-3-phénoxybenzènesulfonamide ;
- N-[[5-(4-bromophényl)-4-(2-chlorophényl)pyrimidin-2-yl]méthyl]-4-(trifluorométhyl)benzènesulfonamide ;
ainsi que leur hydrate ou leur solvat.

8. Procédé de préparation des composés de formule (I) selon la revendication 1
**caractérisé en ce que :**
on traite un composé de formule : 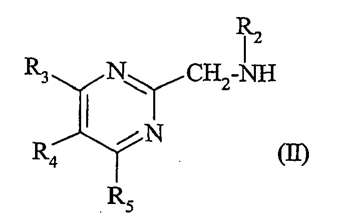
dans laquelle R₂, R₃, R₄ et R₅ sont tels que définis pour un composé de formule (I) à la revendication 1 :
- soit par un acide ou un dérivé fonctionnel de cet acide de formule :
HOOC-R₁ (III)
dans laquelle R₁ est tel que défini pour un composé de formule (I) à la revendication 1, lorsqu'on doit préparer un composé de formule (I) dans laquelle - X- représente un groupe -CO- ;
- soit par un halogénure de sulfonyle de formule :
Hal-SO₂-R₁ (IV)
dans laquelle R₁ est tel que défini pour un composé de formule (I) à la revendication 1, et Hal représente un atome d'halogène, lorsqu'on doit préparer un composé de formule (I) dans laquelle -X- représente un groupe -SO₂- ;
- soit par un halogénoformiate de formule :
HalCOOAr (V)
dans laquelle Hal représente un atome d'halogène et Ar représente un phényle ou un 4-nitrophényle pour obtenir un composé intermédiaire de formule : 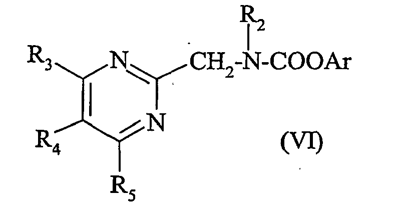 dans laquelle R₂, R₃, R₄ et R₅ sont tels que définis pour un composé de formule (I) à la revendication 1, que l'on fait réagir ensuite avec une amine de formule :
HN(R₆)R₁ (VII)
dans laquelle R₁ et R₆ sont tels que définis pour un composé de formule (I) à la revendication 1, lorsqu'on doit préparer un composé de formule (I) dans laquelle - X- représente un groupe -CON(R₆)-.

9. Médicament, **caractérisé en ce qu'**il comprend un composé de formule (I) selon l'une quelconque des revendications 1 à 7, ou un hydrate ou un solvat du composé de formule (I).

10. Composition pharmaceutique, **caractérisée en ce qu'**elle comprend un composé de formule (I) selon l'une quelconque des revendications 1 à 7, ou un hydrate ou un solvat de ce composé, ainsi qu'au moins un excipient pharmaceutiquement acceptable.

11. Utilisation d'un composé de formule (I) selon l'une quelconque des revendications 1 à 7 pour la préparation d'un médicament destiné au traitement et à la prévention des troubles de l'appétit, des troubles métaboliques, des troubles gastro-intestinaux, des phénomènes inflammatoires, des maladies du système immunitaire, des troubles psychotiques, de la dépendance alcoolique, de la dépendance nicotinique.

12. Utilisation selon la revendication 11 **caractérisée en ce que** les maladies sont les désordres psychiatriques, la dépendance et le sevrage à une substance, les troubles cognitifs, les troubles de l'attention et de la vigilence, et les maladies neurodégénératives aiguës et chroniques.

13. Utilisation selon la revendication 11 **caractérisée en ce que** les maladies sont les troubles du métabolisme, les troubles de l'appétance, les troubles de l'appétit, l'obésité, le diabète de type II, le syndrome métabolique, la dyslipidémie.

14. Utilisation selon la revendication 11 **caractérisée en ce que** les maladies sont la douleur, la douleur neuropathique, la douleur induite par le traitement anticancéreux.

15. Utilisation selon la revendication 11 **caractérisée en ce que** les maladies sont les troubles gastro-intestinaux, les vomissements, les troubles diarrhéiques, l'ulcère, les maladies du foie.

16. Utilisation selon la revendication 11 **caractérisée en ce que** les maladies sont les maladies du système immunitaire, arthrite rhymatoïde, la démyélinisation, la sclérose en plaque, les maladies inflammatoires.

17. Utilisation selon la revendication 11 **caractérisée en ce que** les maladies sont les maladies d'Alzheimer, de Parkinson, la schizophrénie, les troubles cognitifs, le diabète, l'obésité, le syndrome métabolique et le sevrage tabagique.

## Claims

1. Compound corresponding to formula (I): 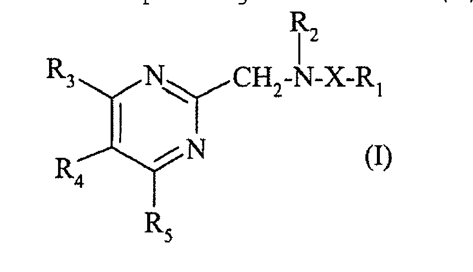 in which:
- X represents a group 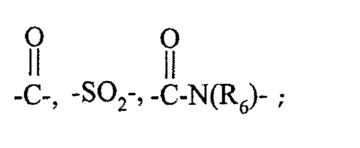
- R₁ represents:
. a (C₁-C₁₂)-alkyl, unsubstituted or substituted one or more times with substituents selected independently from a fluorine atom, a hydroxyl, a (C₁-C₄)-alkoxy, a (C₁-C₄)-alkylthio, a phenoxy, a trifluoromethoxy radical, a difluoromethoxy radical, a difluoromethylthio radical, a trifluoromethylthio radical;
. a non-aromatic (C₃-C₁₂) carbocyclic radical, unsubstituted or substituted one or more times with substituents selected independently from a (C₁-C₄)-alkyl, a (C₁-C₄)-alkoxy, a fluorine atom, a hydroxyl, a trifluoromethyl radical, a trifluoromethoxy radical, a (C₁-C₄)-alkylthio;
. a (C₃-C₇)-cycloalkylmethyl, unsubstituted or substituted one or more times on the carbocycle with substituents selected independently from a (C₁-C₄)-alkyl, a (C₁-C₄)-alkoxy, a fluorine atom, a hydroxyl, a trifluoromethyl radical, a trifluoromethoxy radical;
. a phenyl, unsubstituted or substituted one or more times with substituents selected independently from a halogen atom, a hydroxyl, an Alk group, an OAlk group, a methylenedioxy, a (C₁-C₄)-alkylamino, a di-(C₁-C₄)-alkylamino, a cyano, a nitro, an S(O)ₙAlk group, an OS(O)ₙAlk group, a (C₁-C₄)-alkylcarbonyl group; or from a phenyl, phenoxy, pyrrolyl, imidazolyl, pyridyl or pyrazolyl radical, said radical being unsubstituted or substituted one or more times with a (C₁-C₄)-alkyl;
. a benzyl, unsubstituted or substituted one or more times on the phenyl with substituents selected independently from a halogen atom, an Alk group, a hydroxyl, an OAlk group, a methylenedioxy, an S(O)ₙAlk group, an OS(O)ₙAlk group and unsubstituted or substituted on alpha by one or two similar or dissimilar groups selected from a (C₁-C₄)-alkyl, a (C₃-C₇)-cycloalkyl;
. a phenethyl, unsubstituted or substituted one or more times on the phenyl with substituents selected independently from a halogen atom, a (C₁-C₄)-alkyl, a (C₁-C₄)-alkoxy, a trifluoromethyl radical, a trifluoromethoxy radical;
. a benzhydryl; a benzhydrylmethyl;
. a 1,2,3,4-tetrahydronaphthalenyl, unsubstituted or substituted one or more times with a (C₁-C₄)-alkyl;
. an aromatic heterocyclic radical selected from pyrrolyl, imidazolyl, furyl, thienyl, pyrazolyl, oxazolyl, pyridyl, indolyl, said radical being unsubstituted or substituted one or more times with substituents selected independently from a halogen atom or a (C₁-C₄)-alkyl, a (C₁-C₄)-alkoxy, a trifluoromethyl radical, a trifluoromethoxy radical, a cyano, a nitro, a (C₁-C₄)-alkylthio;
- R₂ represents a hydrogen atom or a (C₁-C₃)-alkyl;
- R₃ represents a hydrogen atom or a (C₁-C₅)-alkyl;
- R₄ represents a phenyl, unsubstituted or substituted one or more times with substituents selected independently from a halogen atom, Alk group, OAlk group, S(O)ₙAlk group or OS(O)ₙAlk group;
- R₅ represents a phenyl, unsubstituted or substituted one or more times with substituents selected independently from a halogen atom, Alk group, OAlk group, S(O)ₙAlk group or OS(O)ₙAlk group;
- R₆ represents a hydrogen atom or a (C₁-C₃)-alkyl;
- n represents 0, 1 or 2;
- Alk represents a (C₁-C₄)-alkyl, unsubstituted or substituted one or more times with a fluorine atom; with the proviso that R₄ and R₅ do not simultaneously represent a phenyl substituted with a (C₁-C₄)-alkoxy; as well as their hydrate or their solvate.

2. Compound according to Claim 1 of formula (IA) in which -X- represents a -CO- radical and the substituents R₁ to R₅ are as defined for the compounds of formula (I) in Claim 1; as well as its hydrates or its solvates.

3. Compound according to Claim 1 of formula (IB) in which -X- represents an -SO₂- radical and the substituents R₁ to R₅ are as defined for the compounds of formula (I) in Claim 1; as well as its hydrates or its solvates.

4. Compound according to Claim 1 of formula (IC) in which -X- represents a -CON(R₆)- radical and the substituents R₁ to R₆ are as defined for the compounds of formula (I) in Claim 1; as well as its hydrates or its solvates.

5. Compound of formula (I) according to Claim 1 in which:
- X represents a -CO- group, an -SO₂- group, a
- CON(R₆)- group;
- R₁ represents:
. a 1-ethylpropyl; a 1-propylbutyl;
. a cyclopentyl; a cyclohexyl; a cycloheptyl; a 2-norbornyl;
. a phenyl; a 4-bromophenyl; a 3-chlorophenyl; a 4-chlorophenyl; a 2-fluorophenyl; a 3-fluorophenyl; a 3,5-difluorophenyl; a 4-*tert*-butylphenyl; a 3,5-dimethylphenyl; a 3-methoxyphenyl; a 4-(diethylamino)phenyl; a 3-(trifluoromethyl)phenyl; a 4-(trifluoromethyl)phenyl; a 2-chloro-4-(trifluoromethyl)phenyl; a 2-nitro-4-(trifluoromethyl)phenyl; a 2-bromo-4-(trifluoromethyl)phenyl; a 2-fluoro-5-(trifluoromethyl)phenyl; a 4-fluoro-3-(trifluoromethyl)phenyl; a 2-chloro-5-(trifluoromethyl)phenyl; a 4-(difluoromethoxy)phenyl; a biphenyl-2-yl; a 3-phenoxyphenyl; a 4-phenoxyphenyl; a 4-(1*H*-pyrrol-1-yl)phenyl;
. a 3-(trifluoromethyl)benzyl;
. a benzhydrylmethyl;
. a 2-thienyl; a 5-chloro-2-thienyl; a 5-bromo-2-furyl; a 3-*tert*-butyl-1-ethyl-1*H*-pyrazol-5-yl; a 1*H-*indol-2-yl; a 1-methyl-1*H*-indol-2-yl;
- R₂ represents a hydrogen atom;
- R₃ represents a hydrogen atom or a methyl;
- R₄ represents a 4-bromophenyl; a 4-chlorophenyl; a 4-methoxyphenyl;
- R₅ represents a 2,4-dichlorophenyl;
- R₆ represents a hydrogen atom; as well as their hydrate or their solvate.

6. Compound of formula (I) according to Claim 1 in which:
- X represents a -CO- or -SO₂- group;
- R₁ represents:
. a 3-(trifluoromethyl)phenyl; a 4-(trifluoromethyl)phenyl; a 2-chloro-4-(trifluoromethyl)phenyl; a 3-phenoxyphenyl; a 4-(1*H-*pyrrol-1-yl)phenyl;
. a benzhydrylmethyl;
- R₂ represents a hydrogen atom;
- R₃ represents a hydrogen atom or a methyl;
- R₄ represents a 4-bromophenyl or a 4-methoxyphenyl;
- R₅ represents a 2,4-dichlorophenyl; as well as their hydrate or their solvate.

7. Compound of formula (I) according to Claim 1 selected from:
- N-[[5-(4-bromophenyl)-4-(2,4-dichlorophenyl)pyrimidin-2-yl]methyl]-3-(trifluoromethyl)benzenesulphonamide;
- N-[[5-(4-bromophenyl)-4-(2,4-dichlorophenyl)pyrimidin-2-yl]methyl]-4-(trifluoromethyl)benzenesulphonamide;
- N-[[4-(2,4-dichlorophenyl)-5-(4-methoxyphenyl)pyrimidin-2-yl]methyl]-4-(1*H*-pyrrol-1-yl)benzamide;
- N-[[5-(4-bromophenyl)-4-(2,4-dichlorophenyl)pyrimidin-2-yl]methyl]-3,3-diphenylpropanamide;
- N-[[5-(4-bromophenyl)-4-(2,4-dichlorophenyl)-6-methylpyrimidin-2-yl]methyl]-3,3-diphenylpropanamide;
- N-[[5-(4-bromophenyl)-4-(2,4-dichlorophenyl)-6-methylpyrimidin-2-yl]methyl]-3-(trifluoromethyl)benzenesulphonamide;
- N-[[5-(4-bromophenyl)-4-(2,4-dichlorophenyl)-6-methylpyrimidin-2-yl]methyl]-4-(trifluoromethyl)benzenesulphonamide;
- N-[[5-(4-bromophenyl)-4-(2,4-dichlorophenyl)-6-methylpyrimidin-2-yl]methyl]-2-chloro-4-(trifluoromethyl)benzenesulphonamide;
- N-[[5-(4-bromophenyl)-4-(2,4-dichlorophenyl)-6-methylpyrimidin-2-yl]methyl]-3-phenoxybenzenesulphonamide;
as well as their hydrate or their solvate.

8. Method of preparation of the compounds of formula (I) according to Claim 1, **characterized in that:**
a compound of formula: 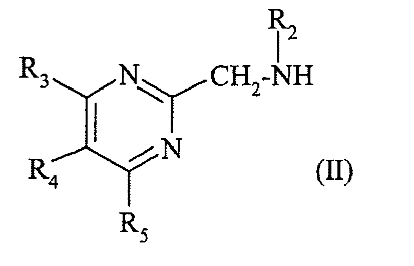
in which R₂, R₃, R₄ and R₅ are as defined for a compound of formula (I) in Claim 1 is treated
- either with an acid or a functional derivative of that acid of formula:
HOOC-R₁ (III)
in which R₁ is as defined for a compound of formula (I) in Claim 1, when it is necessary to prepare a compound of formula (I) in which -X- represents a -CO- group;
- or with a sulphonyl halide of formula:
Hal-SO₂-R₁ (IV)
in which R₁ is as defined for a compound of formula (I) in Claim 1, and Hal represents a halogen atom, preferably chlorine, when it is necessary to prepare a compound of formula (I) in which -X- represents an -SO₂-group;
- or with a haloformate of formula:
HalCOOAr (V)
in which Hal represents a halogen atom and Ar represents a phenyl or a 4-nitrophenyl to obtain an intermediate of formula: 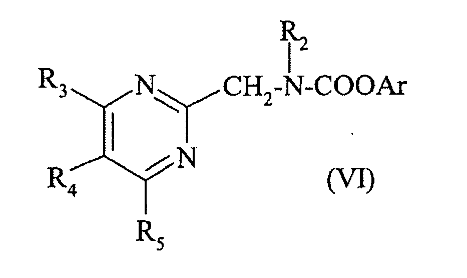 in which R₂, R₃, R₄ and R₅ are as defined for a compound of formula (I) in Claim 1, which is then reacted with an amine of formula:
HN(R₆)R₁ (VII)
in which R₁ and R₆ are as defined for a compound of formula (I) in Claim 1, when it is necessary to prepare a compound of formula (I) in which -X- represents a -CON(R₆)- group.

9. Medicinal product, **characterized in that** it comprises a compound of formula (I) according to any one of the Claims 1 to 7, or a hydrate or a solvate of the compound of formula (I).

10. Pharmaceutical composition, **characterized in that** it comprises a compound of formula (I) according to any one of the Claims 1 to 7, or a hydrate or a solvate of that compound, as well as at least one pharmaceutically acceptable excipient.

11. Use of a compound of formula (I) according to any one of the Claims 1 to 7 for the preparation of a medicinal product intended for the treatment and prevention of disorders of appetite, metabolic disorders, gastrointestinal disorders, inflammatory phenomena, immune system diseases, psychotic disorders, alcohol dependence, and nicotine dependence.

12. Use according to Claim 11, **characterized in that** the diseases are psychiatric disorders, substance dependence and withdrawal, cognitive disorders, disorders of attention and vigilance, and acute and chronic neurodegenerative diseases.

13. Use according to Claim 11, **characterized in that** the diseases are disorders of metabolism, disorders of craving, disorders of appetite, obesity, type II diabetes, metabolic syndrome, and dyslipidaemia.

14. Use according to Claim 11, **characterized in that** the diseases are pain, neuropathic pain, and pain induced by anticancer treatment.

15. Use according to Claim 11, **characterized in that** the diseases are gastrointestinal disorders, vomiting, diarrhoea, ulcers, and liver diseases.

16. Use according to Claim 11, **characterized in that** the diseases are immune system diseases, rheumatoid arthritis, demyelinization, multiple sclerosis, and inflammatory diseases.

17. Use according to Claim 11, **characterized in that** the diseases are Alzheimer's disease, Parkinson's disease, schizophrenia, cognitive disorders, diabetes, obesity, metabolic syndrome and tobacco withdrawal.

## Patentansprüche

1. Verbindung der Formel (I): 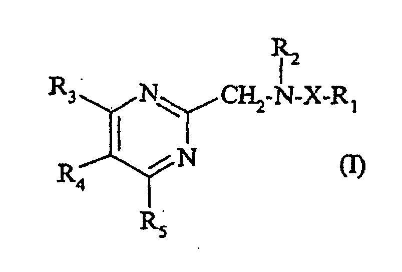 worin:
- X für eine Gruppe 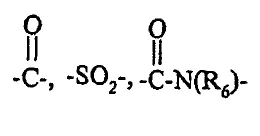 steht;
- R₁ für:
. (C₁-C₁₂)-Alkyl, das gegebenenfalls ein- oder mehrfach durch unabhängig voneinander unter einem Fluoratom, Hydroxyl, (C₁-C₄)-Alkoxy, (C₁-C₄)-Alkylthio, Phenoxy, einem Trifluormethoxyrest, einem Difluormethoxyrest, einem Difluoromethylthiorest und einem Trifluormethylthiorest ausgewählte Substituenten substituiert ist;
. einen nichtaromatischen carbocyclischen (C₃-C₁₂)-Rest, der gegebenenfalls ein- oder mehrfach durch unabhängig voneinander unter (C₁-C₄)-Alkyl, (C₁-C₄)-Alkoxy, einem Fluoratom, Hydroxyl, einem Trifluormetyhlrest, einem Trifluormethoxyrest und (C₁-C₄)-Alkylthio ausgewählte Substituenten substituiert ist;
. (C₃-C₇)-Cycloalkylmethyl, das gegebenenfalls am Carbocyclus ein- oder mehrfach durch unabhängig voneinander unter (C₁-C₄)-Alkyl, (C₁-C₄)-Alkoxy, einem Fluoratom, Hydroxyl, einem Trifluormethylrest und einem Trifluormethoxyrest ausgewählte Substituenten substituiert ist;
. Phenyl, das gegebenenfalls ein- oder mehrfach durch unabhängig voneinander unter einem Halogenatom, Hydroxyl, einer Alk-Gruppe, einer OAlk-Gruppe, Methylendioxy, (C₁-C₄)-Alkylamino, Di-(C₁-C₄)-alkylamino, Cyano, Nitro, einer S(O)ₙAlk-Gruppe, einer OS(O)ₙAlk-Gruppe, einer (C₁-C₄)-Alkylcarbonylgruppe oder unter einem Phenyl-, Phenoxy-, Pyrrolyl-, Imidazolyl-, Pyridyl- oder Pyrazolylrest, der gegebenenfalls ein- oder mehrfach durch (C₁-C₄)-Alkyl substituiert ist, ausgewählte Substituenten substituiert ist;
. Benzyl, das gegebenenfalls am Phenylring ein-oder mehrfach durch unabhängig voneinander unter einem Halogenatom, einer Alk-Gruppe, Hydroxyl, einer OAlk-Gruppe, Methylendioxy, einer S(O)ₙAlk-Gruppe und einer OS(O)ₙAlk-Gruppe ausgewählte Substituenten substituiert ist und gegebenenfalls in alpha-Position durch eine oder zwei Gruppen, die gleich oder verschieden sind und unter (C₁-C₄)-Alkyl und (C₃-C₇)-Cycloalkyl ausgewählt sind, substituiert ist;
. Phenethyl, das gegebenenfalls am Phenylring ein- oder mehrfach durch unabhängig voneinander unter einem Halogenatom, (C₁-C₄)-Alkyl, (C₁-C₄)-Alkoxy, einem Trifluormethylrest und einem Trifluormethoxyrest ausgewählte Substituenten substituiert ist;
. Benzhydryl; Benzhydrylmethyl;
. 1,2,3,4-Tetrahydronaphthalinyl, das gegebenenfalls ein- oder mehrfach durch (C₁-C₄)-Alkyl substituiert ist;
. einen aromatischen heterocyclischen Rest, der unter Pyrrolyl, Imidazolyl, Furyl, Thienyl, Pyrazolyl, Oxazolyl, Pyridyl und Indolyl ausgewählt ist und gegebenenfalls ein- oder mehrfach durch unabhängig voneinander unter einem Halogenatom oder (C₁-C₄)-Alkyl, (C₁-C₄)-Alkoxy, einem Trifluormethylrest, einem Trifluormethoxyrest, Cyano, Nitro und (C₁-C₄)-Alkylthio ausgewählte Substituenten substituiert ist; steht;
- R₂ für ein Wasserstoffatom oder (C₁-C₃)-Alkyl steht;
- R₃ für ein Wasserstoffatom oder (C₁-C₅)-Alkyl steht;
- R₄ für Phenyl, das gegebenenfalls ein- oder mehrfach durch unabhängig voneinander unter einem Halogenatom, einer Alk-Gruppe, einer OAlk-Gruppe, einer S(O)ₙAlk-Gruppe und einer OS(O)ₙAlk-Gruppe ausgewählte Substituenten substituiert ist, steht;
- R₅ für Phenyl, das gegebenenfalls ein- oder mehrfach durch unabhängig voneinander unter einem Halogenatom, einer Alk-Gruppe, einer OAlk-Gruppe, einer S(O)ₙAlk-Gruppe und einer OS(O)ₙAlk-Gruppe ausgewählte Substituenten substituiert ist, steht;
- R₆ für ein Wasserstoffatom oder (C₁-C₃)-Alkyl steht;
- n für 0, 1 oder 2 steht;
- Alk für (C₁-C₄)-Alkyl, das gegebenenfalls ein- oder mehrfach durch ein Fluoratom substituiert ist, steht;
mit der Maßgabe, daß R₄ und R₅ nicht gleichzeitig für durch (C₁-C₄)-Alkoxy substituiertes Phenyl stehen;
sowie ihr Hydrat oder ihr Solvat.

2. Verbindung nach Anspruch 1 der Formel (IA), worin
- X- für einen -CO-Rest steht und die Substituenten R₁ bis R₅ die für Verbindungen der Formel (I) in Anspruch 1 angegebene Bedeutung besitzen; sowie ihre Hydrate oder ihre Solvate.

3. Verbindung nach Anspruch 1 der Formel (IB), worin
- X- für einen -SO₂-Rest steht und die Substituenten R₁ bis R₅ die für Verbindungen der Formel (I) in Anspruch 1 angegebene Bedeutung besitzen; sowie ihre Hydrate oder ihre Solvate.

4. Verbindung nach Anspruch 1 der Formel (IC), worin
- X- für einen -CON(R₆)-Rest steht und die Substituenten R₁ bis R₆ die für Verbindungen der Formel (I) in Anspruch 1 angegebene Bedeutung besitzen; sowie ihre Hydrate oder ihre Solvate.

5. Verbindung der Formel (I) nach Anspruch 1, worin:
- X für eine -CO-Gruppe, eine -SO₂-Gruppe oder eine
- CON(R₆)-Gruppe steht;
- R₁ für:
. 1-Ethylpropyl; 1-Propylbutyl;
. Cyclopentyl; Cyclohexyl; Cycloheptyl; 2-Norbornyl;
. Phenyl; 4-Bromphenyl; 3-Chlorphenyl; 4-Chlorphenyl; 2-Fluorphenyl; 3-Fluorphenyl; 3,5-Difluorphenyl; 4-*tert*.-Butylphenyl; 3,5-Dimethylphenyl; 3-Methoxyphenyl; 4-(Diethylamino)-phenyl; 3-(Trifluormethyl)phenyl; 4-(Trifluormethyl)phenyl; 2-Chlor-4-(trifluormethyl)phenyl; 2-Nitro-4-(trifluormethyl)phenyl; 2-Brom-4-(trifluormethyl)phenyl; 2-Fluor-5-(trifluormethyl)-phenyl; 4-Fluor-3-(trifluoromethyl)phenyl; 2-Chlor-5-(trifluormethyl)phenyl; 4-(Difluor-methoxy)phenyl; Biphenyl-2-yl; 3-Phenoxyphenyl; 4-Phenoxyphenyl; 4-(1*H*-Pyrrol-1-yl)phenyl;
. 3-(Trifluormethyl)benzyl;
. Benzhydrylmethyl;
. 2-Thienyl; 5-Chlor-2-thienyl; 5-Brom-2-furyl; 3-*tert*.-Butyl-1-ethyl-1*H*-pyrazol-5-yl; 1*H*-Indol-2-yl; 1-Methyl-1*H*-indol-2-yl steht;
- R₂ für ein Wasserstoffatom steht;
- R₃ für ein Wasserstoffatom oder Methyl steht;
- R₄ für 4-Bromphenyl, 4-Chlorphenyl oder 4-Methoxyphenyl steht;
- R₅ für 2,4-Dichlorphenyl oder 2-Chlorphenyl steht;
- R₆ für ein Wasserstoffatom steht;
sowie ihr Hydrat oder ihr Solvat.

6. Verbindung der Formel (I) nach Anspruch 1, worin:
- X für eine -CO- oder -SO₂-Gruppe steht;
- R₁ für:
. 3-(Trifluormethyl)phenyl; 4-(Trifluormethyl)-phenyl; 2-Chlor-4-(trifluormethyl)phenyl; 3-Phenoxyphenyl; 4-(1*H*-Pyrrol-1-yl)phenyl;
. Benzhydrylmethyl
steht;
- R₂ für ein Wasserstoffatom steht;
- R₃ für ein Wasserstoffatom oder Methyl steht;
- R₄ für 4-Bromphenyl oder 4-Methoxyphenyl steht;
- R₅ für 2,4-Dichlorphenyl oder 2-Chlorphenyl steht;
sowie ihr Hydrat oder ihr Solvat.

7. Verbindung der Formel (I) nach Anspruch 1, ausgewählt unter:
- N-[[5-(4-Bromphenyl)-4-(2,4-dichlorphenyl)-pyrimidin-2-yl]methyl]-3-(trifluormethyl)-benzolsulfonamid;
- N-[[5-(4-Bromphenyl)-4-(2,4-dichlorphenyl)-pyrimidin-2-yl]methyl]-4-(trifluormethyl)-benzolsulfonamid;
- N-[[4-(2,4-Dichlorphenyl)-5-(4-methoxyphenyl)-pyrimidin-2-yl]methyl]-4-(1*H*-pyrrol-1-yl)benzamid;
- N-[[5-(4-Bromphenyl)-4-(2,4-dichlorphenyl)-pyrimidin-2-yl]methyl]-3,3-diphenylpropanamid;
- N-[[5-(4-Bromphenyl)-4-(2,4-dichlorphenyl)-6-methylpyrimidin-2-yl]methyl]-3,3-diphenyl-propanamid;
- N-[[5-(4-Bromphenyl)-4-(2,4-dichlorphenyl)-6-methylpyrimidin-2-yl]methyl]-3-(trifluormethyl)benzolsulfonamid;
- N-[[5-(4-Bromphenyl)-4-(2,4-dichlorphenyl)-6-methylpyrimidin-2-yl]methyl]-4-(trifluormethyl)benzolsulfonamid;
- N-[[5-(4-Bromphenyl)-4-(2,4-dichlorphenyl)-6-methylpyrimidin-2-yl]methyl]-2-chlor-4-(trifluormethyl)benzolsulfonamid;
- N-[[5-(4-Bromphenyl)-4-(2,4-dichlorphenyl)-6-methylpyrimidin-2-yl]methyl]-3-phenoxybenzol-sulfonamid;
- N-[[5-(4-Bromphenyl)-4-(2-chlorphenyl)-pyrimidin-2-yl]methyl]-4-(trifluormethyl)-benzolsulfonamid;
sowie ihr Hydrat oder ihr Solvat.

8. Verfahren zur Herstellung der Verbindungen der Formel (I) nach Anspruch 1, **dadurch gekennzeichnet, daß** man:
eine Verbindung der Formel: 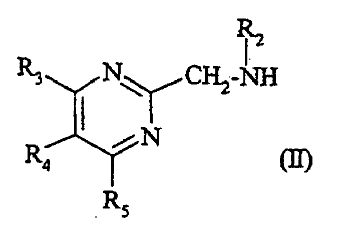
worin R₂, R₃, R₄ und R₅ die für eine Verbindung der Formel (I) in Anspruch 1 angegebene Bedeutung besitzen,
- entweder mit einer Säure oder einem funktionellen Derivat dieser Säure der Formel:
HOOC-R₁ (III)
worin R¹ die für eine Verbindung der Formel (I) in Anspruch 1 angegebene Bedeutung besitzt, behandelt, wenn eine Verbindung der Formel (I), worin -X- für eine -CO-Gruppe steht, herzustellen ist;
- oder mit einem Sulfonylhalogenid der Formel:
Hal-SO₂-R₁ (IV)
worin R¹ die für eine Verbindung der Formel (I) in Anspruch 1 angegebene Bedeutung besitzt und Hal für ein Halogenatom steht, behandelt, wenn eine Verbindung der Formel (I), worin -X- für eine -SO₂-Gruppe steht, herzustellen ist;
- oder mit einem Halogenameisensäureester der Formel:
HalCOOAr (V)
worin Hal für ein Halogenatom steht und Ar für Phenyl oder 4-Nitrophenyl steht, behandelt, was eine Zwischenverbindung der Formel: 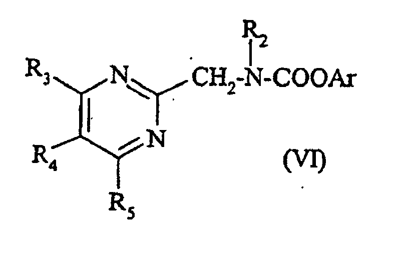
worin R₂, R₃, R₄ und R₅ die für eine Verbindung der Formel (I) in Anspruch 1 angegebene Bedeutung besitzen, ergibt, die man dann mit einem Amin der Formel:
HN(R₆)R₁ (VII)
worin R₁ und R₆ die für eine Verbindung der Formel (I) in Anspruch 1 angegebene Bedeutung besitzen, umsetzt, wenn eine Verbindung der Formel (I), worin -X- für eine -CON(R₆)-Gruppe steht, herzustellen ist.

9. Arzneimittel, **dadurch gekennzeichnet, daß** es eine Verbindung der Formel (I) nach einem der Ansprüche 1 bis 7 oder ein Hydrat oder ein Solvat der Verbindung der Formel (I) enthält.

10. Pharmazeutische Zusammensetzung, **dadurch gekennzeichnet, daß** sie eine Verbindung der Formel (I) nach einem der Ansprüche 1 bis 7 oder ein Hydrat oder ein Solvat der Verbindung der Formel (I) sowie einen pharmazeutisch unbedenklichen Hilfsstoff enthält.

11. Verwendung einer Verbindung der Formel (I) nach einem der Ansprüche 1 bis 7 zur Herstellung eines Arzneimittels zur Behandlung oder Prävention von Appetitstörungen, Stoffwechselstörungen, Magen-Darm-Störungen, Entzündungsphänomenen, Erkrankungen des Immunsystems, psychotischen Störungen, Alkoholabhängigkeit und Nikotinabhängigkeit.

12. Verwendung nach Anspruch 11, **dadurch gekennzeichnet, daß** es sich bei den Erkrankungen um psychotische Störungen, Substanzabhängigkeit und -entwöhnung, kognitive Störungen, Aufmerksamkeits- oder Wachsamkeitsstörungen und akute und chronische neurodegenerative Erkrankungen handelt.

13. Verwendung nach Anspruch 11, **dadurch gekennzeichnet, daß** es sich bei den Erkrankungen um Stoffwechselstörungen, Appetenzstörungen, Appetitstörungen, Obesitas, Typ-II-Diabetes, metabolisches Syndrom und Dyslipidämie handelt.

14. Verwendung nach Anspruch 11, **dadurch gekennzeichnet, daß** es sich bei den Erkrankungen um Schmerzen, neuropathische Schmerzen und durch Antikrebsbehandlung induzierte Schmerzen handelt.

15. Verwendung nach Anspruch 11, **dadurch gekennzeichnet, daß** es sich bei den Erkrankungen um Magen-Darm-Störungen, Erbrechen, Durchfallstörungen, Geschwüre und Lebererkrankungen handelt.

16. Verwendung nach Anspruch 11, **dadurch gekennzeichnet, daß** es sich bei den Erkrankungen um Erkrankungen des Immunsystems, rheumatoide Arthritis, Demyelinisierung, multiple Sklerose und entzündliche Erkrankungen handelt.

17. Verwendung nach Anspruch 11, **dadurch gekennzeichnet, daß** es sich bei den Erkrankungen um Alzheimer-Krankheit, Parkinson-Krankheit, Schizophrenie, kognitive Störungen, Diabetes, Obesitas, metabolisches Syndrom und Tabakentwöhnung handelt.
